# EUROPÄISCHE PATENTSCHRIFT

(11) **EP 1 121 349 B1**
(45) Veröffentlichungstag und Bekanntmachung des Hinweises auf die Patenterteilung: **31.03.2004**
(21) Anmeldenummer: 99947450.5
(22) Anmeldetag: 02.10.1999
(51) Int. Cl.: C07D 285/08, A61K 31/41, C07D 417/12, A61K 31/495, A61K 31/445

(54) **SUBSTITUIERTE THIADIAZOLSULFONAMIDE ALS INTERLEUKIN-1-BETA-HEMMER**
SUBSTITUTED THIADIAZOLSULFONAMIDES USED AS INTERLEUKIN-1-BETA INHIBITORS
THIADIAZOLSULFONAMIDES SUBSTITUES SERVANT D'INHIBITEUR DE L'INTERLEUKINE 1-BETA

(30) Priorität: 16.10.1998 DE 19847823
(43) Veröffentlichungstag der Anmeldung: 08.08.2001
(73) Patentinhaber: Aventis Pharma Deutschland GmbH, 65929 Frankfurt am Main (DE)
(72) Erfinder: THORWART, Werner, D-65239 Hochheim (DE); WEITHMANN, Klaus-Ulrich, D-65719 Hofheim (DE); HÖLDER, Swen, D-60954 Frankfurt am Main (DE)
(86) Internationale Anmeldenummer: PCT/EP1999/007301
(87) Internationale Veröffentlichungsnummer: WO 2000/023430

(56) Entgegenhaltungen:
- US-A- 4 758 578
- YOUSIF N M ET AL: "Synthesis and reactions of some 1,2,4-thiadiazole derivatives for biological evaluation" EGYPT. J. CHEM. (EGJCA3,03670422), Bd. 32, Nr. 5, 1991, Seiten 607-14, XP002124969 Natl. Res. Cent.;Cairo; Egypt (EG) in der Anmeldung erwähnt

## Beschreibung

Die Erfindung betrifft neue substituierte Thiadiazolsulfonamide, Verfahren zu ihrer Herstellung und Verwendung derselben als Arzneimittel.

In den Anmeldungen US 4 758 578, US 4 873 239 und US 4 985 450 werden nur solche Arylthiadiazolsulfonamide beschrieben, die sich zur Freisetzung von am Stickstoff unsubstituierter Sulfonamide eignen und daher insbesondere zur Glaukombehandlung eingesetzt werden können.

Ferner sind einige 1,2,4-Thiadiazol-sulfonamid Derivate mit bakterizider Wirkung bekannt (N.M. Yousif et al., Egypt. J. Chem. 32 (1989) 607-614).

In dem Bestreben wirksamere und besser verträglichere Arzneimittel zur Behandlung rheumatischer Erkrankungen zu entwickeln, nehmen solche Verbindungen an Bedeutung zu, die in der Lage sind, die überschießende oder fehlregulierte Cytokin-Produktion zu beeinflussen (Annual Reports in Medicinal Chemistry Vol. 27, (1992), S 209-218). Besonders das Interleukin-Iβ (IL-1β), als eines der wichtigsten proinflammatorischen Cytokine, ist hier zu beachten. Seine de novo Synthese findet vor allem in den weißen Blutzellen und Makrophagen statt, wobei es zur Entfaltung seiner Wirkung, es zuerst aus inaktivem pro-IL-1β freigesetzt werden muß. Es spielt eine entscheidende Rolle sowohl bei der Steuerung des Immunsystems als auch bei Entzündungsprozessen, und steht somit im ursächlichen Zusammenhang bei der Entstehung von Erkrankungen, wie Rheumatoider Arthritis, Arthrose, verschiedener Schockzustände oder entzündlichen Knochenerkrankungen.

Erfindungsgemäß wurde nun überraschenderweise gefunden, daß bestimmte Thiadiazolsulfonamide starke Inhibitoren der zellulären Interleukin-1β Generierung sind. 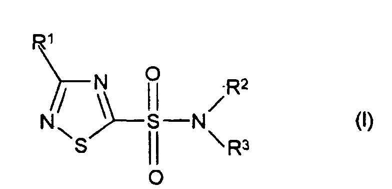 und/oder eine stereoisomere Form der Verbindung der Formel I und/oder ein physiologisch verträgliches Salz der Verbindung der Formel I, wobei
- R¹ für: 1. Phenyl,
2. Phenyl, welches ein- bis dreifach substituiert ist durch
   2.1. -(C₁-C₆)-Alkyl, worin Alkyl gerade, cyclisch oder verzweigt ist,
   2.2. -OH,
   2.3. -O-C(O)-(C₁-C₆)-Alkyl-,
   2.4. -O-(C₁-C₆)-Alkyl,
   2.5. Methylendioxo,
   2.6. Halogen,
   2.7. -CF₃,
   2.8. -CN,
   2.9. -NO₂,
   2.10. -C(O)-OH,
   2.11. -C(O)-O-(C₁-C₆)-Alkyl oder
   2.12. R³-(R⁴ )N-, worin R³ und R⁴ gleich oder verschieden sind und Wasserstoffatom oder (C₁-C₆)-Alkyl- bedeuten, steht, oder
3. einen Heteroaromaten aus der nachfolgenden Gruppe 3.1. bis 3.15., der unsubstituiert oder wie unter 2.1 bis 2.12. beschrieben substituiert ist,
   3.1. Pyrrol,
   3.2. Pyrazol,
   3.3. Imidazol,
   3.4. Triazol,
   3.5. Thiophen,
   3.6. Thiazol,
   3.7. Oxazol,
   3.8. Isoxazol,
   3.9. Pyridin,
   3.10. Pyrimidin,
   3.11. Indol,
   3.12 Benzothiophen,
   3.13. Benzimidazol,
   3.14. Benzoxazol oder
   3.15. Benzothiazol, steht,
- R³ für: 1. Wasserstoffatom oder
2. (C₁-C₆)-Alkyl steht,
- R² für: 1. (C₁-C₇)-Alkyl, worin Alkyl verzweigt oder unverzweigt ist und unsubstituiert ist oder ein- oder zweifach wie oben unter 2.1 bis 2.12. beschrieben substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie oben unter 2.1 bis 2.12. beschrieben substituiert ist,
2. -(C₂-C₁₀)-Alkenyl, worin Alkenyl gerade oder verzweigt ist,
3. (C₃-C₇)-Cycloalkyl, worin Cycloalkyl unsubstituiert ist oder ein- oder zweifach wie oben unter 2.1 bis 2.12. beschrieben substituiert ist,
4. Phenyl, worin Phenyl unsubstituiert oder ein- oder zweifach wie oben unter 2.1. bis 2.12. beschrieben substituiert ist,
5. -(CH₂)ₘ-Phenyl, worin Phenyl unsubstituiert oder ein- oder zweifach wie oben unter 2.1. bis 2.12. beschrieben substituiert ist, m die ganze Zahl 1, 2, 3, 4, 5 oder 6 darstellt, oder ein Wasserstoffatom des -(CH₂)ₘ-Restes durch -OH ersetzt ist,
6. -(CH₂)ₙ-Heteroaryl, worin Heteroaryl wie unter 3.1. bis 3.15. definiert ist und unsubstituiert oder wie oben unter 2.1 bis 2.12. beschrieben substituiert ist und n die ganze Zahl Null, 1, 2, 3, 4, 5 oder 6 darstellt,
7. -(C₂-C₆)-Alkyl-O-R⁶,
   worin R⁶ Wasserstoffatom, Benzyl, Allyl oder (C₁-C₆)-Alkyl darstellt und worin der Alkylrest gerade oder verzweigt ist,
8. -(C₂-C₆)-Alkyl-C(O)-OR⁶, worin R⁶ wie oben definiert ist,
9. -C(R⁸,R⁷)-C(O)-X, worin R⁸ die gleiche Bedeutung wie oben R³ hat, R⁷ den Rest einer α- Aminosäure der Formel II bedeutet 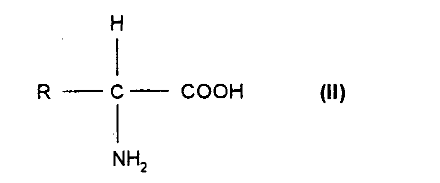 worin R sich von einer natürlichen oder unnatürlichen Aminosäure ableitet, oder R⁸ und R⁷ zusammen mit dem Kohlenstoffatom an dem sie gebunden sind einen 4- bis 7-gliedrigen Ring bilden, und X für -OH, NHR⁶ oder -OR⁶ steht, worin R⁶ wie oben definiert ist, steht oder
10. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
   10.1 (C₁-C₂)-Alkyl,
   10.2 Phenyl,
   10.3 - OH,
   10.4 = O ,
   10.5 -C(O)-O-(C₁-C₆)-Alkyl,
   10.6 -C(O)-Phenyl, worin Phenyl unsubstituiert oder wie oben unter 2.1 bis 2.12 beschrieben substituiert ist,
   10.7 -C(O)-(C₁-C₆)-Alkyl, oder
   10.8 -C-(O)-OR³, worin R³ Wasserstoffatom oder (C₁-C₆)-Alkyl darstellt, und/oder
   10.9 zwei Kohlenstoffatome im Ring Teil eines zusätzlichen Phenylrestes bilden, der unsubstituiert oder wie oben unter 2.1 bis 2.12 beschrieben substituiert ist oder
   worin das Wasserstoffatom in dem gegebenenfalls vorhandenen -NH- im Ring unsubstituiert ist oder durch
   a) (C₁-C₃)-Alkyl,
   b) -C(O)-R³, worin R³ wie oben definiert ist,
   c) -(CH₂)ₒ-Phenyl, worin Phenyl unsubstituiert oder ein- oder zweifach wie unter 2.1 bis 2.12. beschrieben substituiert ist und o die ganze Zahl Null, 1 oder 2 darstellt,
   d) Benzoyl, das unsubstituiert oder wie oben unter 2. 1bis 2.12 beschrieben substituiert ist oder durch
   e) -C(O)-O-(C₁-C₆)-Alkyl ersetzt ist,
   wobei die Verbindung, worin R¹ für Phenyl, R² für Phenyl und R³ für Wasserstoffatom stehen, ausgeschlossen sind.

Bevorzugt ist eine Verbindung der Formel I, wobei
- R¹ für: 1. Phenyl oder
2. Phenyl, welches einfach substituiert ist durch
   2.1 (C₁-C₆)-Alkyl, worin Alkyl gerade oder verzweigt ist,
   2.2 Halogen ,
   2.3 -O-(C₁-C₃)Alkyl oder
   2.4 -N-R³-(R⁴), worin R³ und R⁴ gleich oder verschieden sind und Wasserstoffatom oder (C₁-C₆)-Alkyl- bedeuten, steht
- R³ für: Wasserstoffatom oder Methyl steht,
- R² für: 1. (C₁-C₄)-Alkyl, worin Alkyl verzweigt oder unverzweigt ist und einfach wie unter 2.1 bis 2.12. beschrieben oder durch Phenyl substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie unter 2.1 bis 2.12. beschrieben substituiert ist,
2. Cyclohexyl,
3. -C(R⁸,R⁷)-C(O)-X, worin R⁸ Wasserstoffatom bedeutet, R⁷ den Rest einer α- Aminosäure bedeutet, oder R⁸ und R⁷ zusammen mit dem Kohlenstoffatom an dem sie gebunden sind einen 4- bis 7-gliedrigen Ring bilden, und X für -OH oder -OR⁶ steht, worin R⁶ Benzyl oder (C₁-C₃)-Alkyl bedeutet, steht oder
4. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
   4.1 Phenyl, unsubstituiert oder substituiert durch Halogen,
   4.2 -OH,
   4.3 = O,
   4.4 -C(O)-OH,
   4.5 -C(O)-(C₁-C₂)-Alkyl
   4.6 (C₁-C₃)-Alkyl oder
   4.7 -(O)-OR³ substituiert ist und worin R³ Wasserstoffatom oder (C₁-C₆)-Alkyl darstellt,
      oder worin das Wasserstoffatom in dem gegebenenfalls vorhandenen - NH- im Ring unsubstituiert ist oder durch
      a) (C₁-C₃)-Alkyl,
      b) -C(O)-R³, worin R³ Wasserstoffatom oder (C₁-C₆)-Alkyl darstellt,
      c) Phenyl-(CH₂)ₒ-, worin Phenyl unsubstituiert oder ein- oder zweifach wie unter 2.1 bis 2.12. beschrieben substituiert ist und o die ganze Zahl Null, 1 oder 2 darstellt, oder
      d) C(O)-O-(C₁-C₃)-Alkyl ersetzt ist, und/oder
   4.8 zwei Kohlenstoffatome im Ring Teil eines zusätzlichen Phenylrestes bilden.

Insbesondere bevorzugt ist eine Verbindung der Formel I, wobei
- R¹ für: 1. Phenyl oder
2. Phenyl, welches einfach substituiert ist durch
   2.1 (C₁-C₃)-Alkyl, worin Alkyl gerade oder verzweigt ist,
   2.2 Chlor oder
   2.3 -O-Methyl steht,
- R³ für: Wasserstoffatom oder Methyl steht,
- R² für: 1. (C₁-C₄)-Alkyl, worin Alkyl verzweigt oder unverzweigt ist und einfach durch Phenyl, C(O)-OH, Halogen, Piperidin oder Cyclohexyl substituiert ist, worin Piperidin oder Cyclohexyl unsubstituiert ist oder ein- oder zweifach durch Halogen oder C(O)-OH substituiert ist,
2. Cyclohexyl,
3. -C(R⁸,R⁷)-C(O)-X, worin R⁸ Wasserstoffatom bedeutet, R⁷ Methyl bedeutet und X für -OH oder -OR⁶ steht, worin R⁶ Benzyl oder (C₁-C₃)-Alkyl bedeutet, steht oder
4. R² und R³ zusammen mit dem Stickstoff an dem sie gebunden sind einen Morpholin-, Piperidin-, Piperazin- oder Tetrahydroisochinolin-3-carbonsäurerest bilden, und worin die Kohlenstoffatome im Ring unsubstituiert sind oder ein- oder zweifach substituiert sind durch
   4.1 Phenyl, substituiert durch Chlor oder Fluor,
   4.2 Methyl,
   4.3 = O,
   4.4 -C(O)-OH oder
   4.5 -C(O)-Methyl,
   oder worin das Wasserstoffatom in dem gegebenenfalls vorhandenen - NH- im Ring unsubstituiert ist oder durch
   a) Methyl,
   b) -C(O)-Methyl,
   c) Phenyl, substituiert durch Chlor, oder
   d) C(O)-O-Ethyl ersetzt ist.

Mit dem Begriff "R² und R³ zusammen mit dem Stickstoff an den sie gebunden sind einen 4- bis 7-gliedrigen Ring bilden, worin gegebenenfalls eines der Kohlenstoffatome durch -O-, -S- oder -NH- ersetzt ist" werden Reste verstanden, die sich beispielsweise von Azetidin, Pyrrolin, Pyrrolidin, Piperidin, Piperazin, Morpholin, Thiomorpholin, Tetrahydroisochinolin oder Tetrahydroisochinolin-3-carbonsäure (TIC) ableiten. Mit dem Begriff "R⁸ und R⁷ zusammen mit dem Kohlenstoff an den sie gebunden sind einen 4-bis 7-gliedrigen Ring bilden" werden Reste verstanden, die sich beispielsweise von Azetidin, Pyrrolin, Pyrrolidin, Piperidin, Piperazin, Morpholin, Thiomorpholin, Tetrahydroisochinolin oder Tetrahydroisochinolin-3-carbonsäure ableiten. Unter dem Begriff "Heteroalkylring" werden Reste verstanden, die sich beispielsweise von Piperidin, Azetidin, Pyrrolidin, Isoxazolidin, Morpholin, Isothiazolidin, Thiomorpholin, Pyrazolidin, Imidazolidin, Piperazin, Pyran oder Thiopyran ableiten. Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffkette geradkettig oder verzweigt ist. Femer können die Alkenylreste auch mehrere Doppelbindungen enthalten. Cyclische Alkylreste sind beispielsweise 3- bis 7-gliedrige Monocyclen wie Cyclopropyl, Cyclobutyl, Clyclopentyl, Cyclohexyl oder Cycloheptyl.

Unter dem Begriff "R⁷ den Rest einer α-Aminosäure bedeutet" werden Reste R der Formel II verstanden, 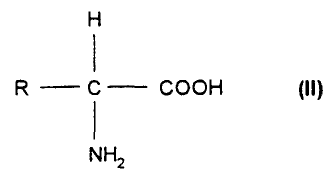 worin R sich von einer natürlichen oder unnatürlichen Aminosäure ableitet. Unter natürliche Aminosäure werden Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure verstanden. Unter unnatürliche Aminosäure werden solche Verbindungen der Formel II verstanden, bei denen beispielsweise der Rest R durch weitere Substituenten in der natürlichen Seitenkette abgeändert wird oder R ein Phenyl, Cycloalkyl, wie Cyclohexyl oder Heteroaryl-(CH₂)ₙ wie unter "R² steht für 5" beschrieben darstellt. Vorzugsweise werden die charakteristischen Reste beispielsweise der folgenden nicht natürlich vorkommenden Aminosäuren 2-Aminoadipinsäure, 2-Aminobuttersäure, 2-Aminoisobuttersäure , 2,3-Diaminopropionsäure, 2,4-Diaminobuttersäure, 1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Aminoheptansäure, Pipecolinsäure, Hydroxylysin, Sarkosin, N-Methylisoleucin, 6-N-Methyllysin, N-Methylvalin, Norvalin, Norleucin, Ornithin, allo-Isoleucin, allo-Threonin, 4-Hydroxyprolin, 3-Hydroxyprolin, allo-Hydroxylysin, 3-(2-Naphtyl)-alanin, 3-(1-Naphtylalanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, Phosphinothricin, 4-Fluorphenylalanin, 3-Fluorphenylalanin, 2-Fluorphenylalanin, 4-Chlorphenylalanin, 4-Nitrophenylalanin, 4-Aminophenylalanin, Cyclohexylalanin, Citrullin, 5-Fluortryptophan, 5-Methoxytryptophan, Methionin-Sulfon, Methionin-Sulfoxid oder NH₂-NH-CONH₂, ggf. substituiert, eingesetzt. Von den natürlichen oder unnatürlichen Aminosäuren werden beide enantiomeren Formen, das Racemat oder eine beliebige Mischung eingesetzt.

Die Erfindung betrifft ferner ein Verfahren zur Herstellung der Verbindung der Formel I und/oder einer stereoisomere Form der Verbindung der Formel I und/oder eines physiologisch verträglichen Salzes der Verbindung der Formel I, das dadurch gekennzeichnet ist, daß man
a) ein Amin der Formel III, 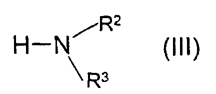 worin R² und R³ wie in Formel I definiert sind, mit einem Sulfonsäurederivat der Formel IV, 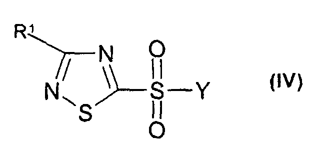 worin R¹ wie in Formel I definiert ist und Y ein Halogenatom, Imidazolyl, oder -OR¹⁰ bedeutet, worin R¹⁰ Wasserstoffatom, (C₁-C₆)-Alkyl, Phenyl oder Benzyl darstellt,
   in Gegenwart einer Base oder gegebenenfalls eines wasserentziehenden Mittels zu einer Verbindung der Formel I umsetzt, oder
b) 5-Mercapto-thiadiazole der Formel V, 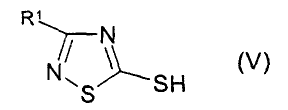 wobei R¹ die oben genannte Bedeutung hat,
   in Gegenwart von Natriumhypochlorit oder Chlor und einem Amin der Formel III in ein Sulfenamine der Formel VI , 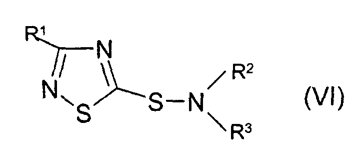 wobei R¹, R² und R³ die obengenannte Bedeutung haben, überführt und die Verbindung der Formel VI in Anwesenheit eines Oxydationsmittels wie Persäuren, insbesondere Peressigsäure, m-Chlorperbenzoesäure oder Wasserstoffperoxyd oder Kaliumpermanganat in die Verbindung Formel I umwandelt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a) oder b) hergestellte Verbindung der Formel I entweder in freier Form isoliert oder im Falle des Vorliegens von sauren oder basischen Gruppen in physiologisch verträgliche Salze umwandelt.

Die Ausgangsstoffe der chemischen Umsetzungen sind bekannt oder lassen sich nach literaturbekannten Methoden leicht herstellen. Als Ausgangsprodukte zur Darstellung eines Sulfonsäurederivats der Formel IV, insbesondere wenn Y ein Chloratom darstellt, dienen 5-Mercaptothiadiazole der Formel V, die üblicherweise durch Einleiten von Chlor in eine Suspension der Verbindung der Formel V in einer Mischung von Essigsäure und Wasser im Verhältnis 1:2 bis 5:1 bei Reaktionstemperaturen von 0 °C bis 30 °C direkt zu dem entsprechenden Sulfonsäurechloriden der Formel IV oxidiert werden.
Die Darstellung einer Verbindung der Formel I nach Verfahrensweg b) erfolgt über die Sulfenamide der Formel VI, die bevorzugt aus einem 5-Mercaptothiadiazol der Formel V und einem Amin der Formel III im wäßrigen Medium mit Natriumhypochlorit bei Reaktionstemperaturen von -5 °C bis 35 °C entstehen. Zur anschließenden Oxidation zu den Verbindungen der Formel I werden bevorzugt Persäuren, wie Peressigsäure, m-Chlorperbenzoesäure, Kaliumpermanganat oder Wasserstoffperoxyd eingesetzt (wie in US 4,985,450 beschrieben).

Die Herstellung physiologisch verträglicher Salze aus zur Salzbildung befähigten Verbindungen der Formel I, einschließlich deren stereoisomere Formen, erfolgt in an sich bekannter Weise. Die gegebenenfalls vorhandenen Säuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindung der Formel I basische Gruppen aufweist, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bemstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder einer gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

IL-1β und seine krankheitsverursachenden Wirkungen sind in der Patentschrift EP 0 607 775, US 5 556 870 und in der dort zitierten Literatur beschrieben. Aufgrund der pharmakologischen Eigenschaften eignen sich die Verbindungen der Formel I und/oder eine stereoisomere Form der Verbindung der Formel I und/oder ein physiologisch verträgliches Salz der Verbindung der Formel I, wobei
- R¹ für: 1. Phenyl,
2. Phenyl, welches ein- bis dreifach substituiert ist durch
   2.1. -(C₁-C₆)-Alkyl, worin Alkyl gerade, cyclisch oder verzweigt ist,
   2.2. -OH,
   2.3. -O-C(O)-(C₁-C₆)-Alkyl-,
   2.4. -O-(C₁-C₆)-Alkyl,
   2.5. Methylendioxo,
   2.6. Halogen,
   2.7. -CF₃,
   2.8. -CN,
   2.9. -NO₂,
   2.10. -C(O)-OH,
   2.11. -C(O)-O-(C₁-C₆)-Alkyl oder
   2.13. R³-(R⁴ )N-, worin R³ und R⁴ gleich oder verschieden sind und Wasserstoffatom oder (C₁-C₆)-Alkyl- bedeuten, steht, oder
3. einen Heteroaromaten aus der nachfolgenden Gruppe 3.1. bis 3.15., der unsubstituiert oder wie unter 2.1 bis 2.12. beschrieben substituiert ist,
   3.1. Pyrrol,
   3.2. Pyrazol,
   3.3. Imidazol,
   3.4. Triazol,
   3.5. Thiophen,
   3.6. Thiazol,
   3.7. Oxazol,
   3.8. Isoxazol,
   3.9. Pyridin,
   3.10. Pyrimidin,
   3.11. Indol,
   3.12 Benzothiophen,
   3.13. Benzimidazol,
   3.14. Benzoxazol oder
   3.15. Benzothiazol, steht,
- R³ für: 1. Wasserstoffatom oder
2. (C₁-C₆)-Alkyl steht,
- R² für: 1. (C₁-C₇)-Alkyl, worin Alkyl verzweigt oder unverzweigt ist und unsubstituiert ist oder ein- oder zweifach wie oben unter 2.1 bis 2.12. beschrieben substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie oben unter 2.1 bis 2.12. beschrieben substituiert ist,
2. -(C₂-C₁₀)-Alkenyl, worin Alkenyl gerade oder verzweigt ist,
3. (C₃-C₇)-Cycloalkyl, worin Cycloalkyl unsubstituiert ist oder ein- oder zweifach wie oben unter 2.1 bis 2.12. beschrieben substituiert ist,
4. Phenyl, worin Phenyl unsubstituiert oder ein- oder zweifach wie oben unter 2.1. bis 2.12. beschrieben substituiert ist,
5. -(CH₂)ₘ-Phenyl, worin Phenyl unsubstituiert oder ein- oder zweifach wie oben unter 2.1. bis 2.12. beschrieben substituiert ist, m die ganze Zahl 1, 2, 3, 4, 5 oder 6 darstellt, oder ein Wasserstoffatom des -(CH₂)ₘ-Restes durch -OH ersetzt ist,
6. -(CH₂)ₙ-Heteroaryl, worin Heteroaryl wie unter 3.1. bis 3.15. definiert ist und unsubstituiert oder wie oben unter 2.1 bis 2.12. beschrieben substituiert ist und n die ganze Zahl Null, 1, 2, 3, 4, 5 oder 6 darstellt,
7. -(C₂-C₆)-Alkyl-O-R⁶,
   worin R⁶ Wasserstoffatom, Benzyl, Allyl oder (C₁-C₆)-Alkyl darstellt und worin der Alkylrest gerade oder verzweigt ist,
8. -(C₂-C₆)-Alkyl-C(O)-OR⁶, worin R⁶ wie oben definiert ist,
9. -C(R⁸,R⁷)-C(O)-X, worin R⁸ die gleiche Bedeutung wie oben R³ hat, R⁷ den Rest einer α- Aminosäure der Formel II mit den oben angegebenen Bedeutungen für R bedeutet, oder R⁸ und R⁷ zusammen mit dem Kohlenstoffatom an dem sie gebunden sind einen 4- bis 7-gliedrigen Ring bilden, und X für -OH, NHR⁶ oder -OR⁶ steht, worin R⁶ wie oben definiert ist, steht oder
10. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
   10.1 (C₁-C₂)-Alkyl,
   10.2 Phenyl,
   10.3 -OH,
   10.4 = O ,
   10.5 -C(O)-O-(C₁-C₆)-Alkyl,
   10.6 -C(O)-Phenyl, worin Phenyl unsubstituiert oder wie oben unter 2.1 bis 2.12 beschrieben substituiert ist,
   10.7 -C(O)-(C₁-C₆)-Alkyl, oder
   10.8 -C-(O)-OR³, worin R³ Wasserstoffatom oder (C₁-C₆)-Alkyl darstellt, und/oder
   10.9 zwei Kohlenstoffatome im Ring Teil eines zusätzlichen Phenylrestes bilden, der unsubstituiert oder wie oben unter 2.1 bis 2.12 beschrieben substituiert ist oder
   worin das Wasserstoffatom in dem gegebenenfalls vorhandenen -NH- im Ring unsubstituiert ist oder durch
   a) (C₁-C₃)-Alkyl,
   b) -C(O)-R³, worin R³ wie oben definiert ist,
   c) -(CH₂)ₒ-Phenyl, worin Phenyl unsubstituiert oder ein- oder zweifach wie unter 2.1 bis 2.12. beschrieben substituiert ist und o die ganze Zahl Null, 1 oder 2 darstellt,
   d) Benzoyl, das unsubstituiert oder wie oben unter 2.1 bis 2.12 beschrieben substituiert ist oder durch
   e) -C(O)-O-(C₁-C₆)-Alkyl ersetzt ist,
zur Herstellung von Arzneimitteln zur Prophylaxe und Therapie von Erkrankungen, die durch IL-1β gekennzeichnet sind oder solchen, an deren Entstehung IL-1β beteiligt ist. Die zu behandelnden Krankheiten umfassen beispielsweise Leukämie, septischer Schock, Hepatitis, HIV-Infektionen oder musculoskeletale Erkrankungen wie Muskelabbau, degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Dazu gehören auch Erkrankungen des Bindegewebes wie Kollagenosen, Periodontalerkrankungen, Wundheilungsstörungen und insbesondere chronische Erkrankungen des Bewegungsapparates wie entzündliche, immunologisch oder stoffwechselbedingte akute und chronische Arthritiden, Arthropathien, rheumatoide Arthritis, Myalgien und Störungen des Knochenstoffwechsels.
Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro) Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glyzerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, können diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel I - Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 400 MHz-Gerät der Firma Bruker aufgenommen worden, in der Regel mit Tetramethylsilan (TMS) als internem Standard und bei Raumtemperatur (RT). Als Lösemittel wurde jeweils DMSO-d₆ verwendet, falls nicht anders vermerkt. Endprodukte werden in der Regel durch massenspektroskopische Methoden (FAB-, ESI-MS) bestimmt. Temperaturangaben in Grad Celsius, RT bedeutet Raumtemperatur (20 °C bis 26 °C). Verwendete Abkürzungen sind entweder erläutert oder entsprechen den üblichen Konventionen.

### Beispiel 16 (S)-2-(3-Phenyl-(1,2,4) thiadiazol-5-sulfonylamino)-propionsäure hergestellt nach Verfahrensvariante a)

Zu einer Lösung von 1,30 g ( 5 mMol) 3-Phenyl-(1,2,4) thiadiazol-5-sulfonylchlorid in 20 ml Toluol wird unter Rühren bei 0°C eine Mischung von 0,72 g ( 5 mMol ) (S)-Alanintert.butylester und 0,86 g ( 7,5 mMol) N-Ethylmorpholin in 20 ml Toluol innerhalb von 30 Minuten zugetropft. Nach 2 stündlichem Rühren wird durch Zugabe von 100 ml 1N-Salzsäure die Mischung sauer gestellt, die organische Phase abgetrennt und die wäßrige Phase mehrmals mit Toluol extrahiert. Trocknen der vereinigten organischen Phasen über Natriumsulfat und Einengen des Filtrats unter verminderten Druck ergibt einen öligen Rückstand an rohem tert.- Butylester. Zur Freisetzung der Säure wird der Ester in 20 ml Methylenchlorid aufgenommen und mit 3 ml Trifluoressigsäure bei RT 4 Stunden behandelt. Danach wird unter verminderten Druck die Mischung zur Trockene eingeengt und der Rückstand aus Toluol/ Methylcyclohexan (1:4) umkristallisiert.
Ausbeute: 1,05 g weiße Nadeln ( 67 % der Theorie)
Schmelzpunkt: 138-139 °C

Die in der folgenden Tabelle 1 genannten Verbindungen wurden analog zu dem vorhergehenden Beispiel hergestellt.

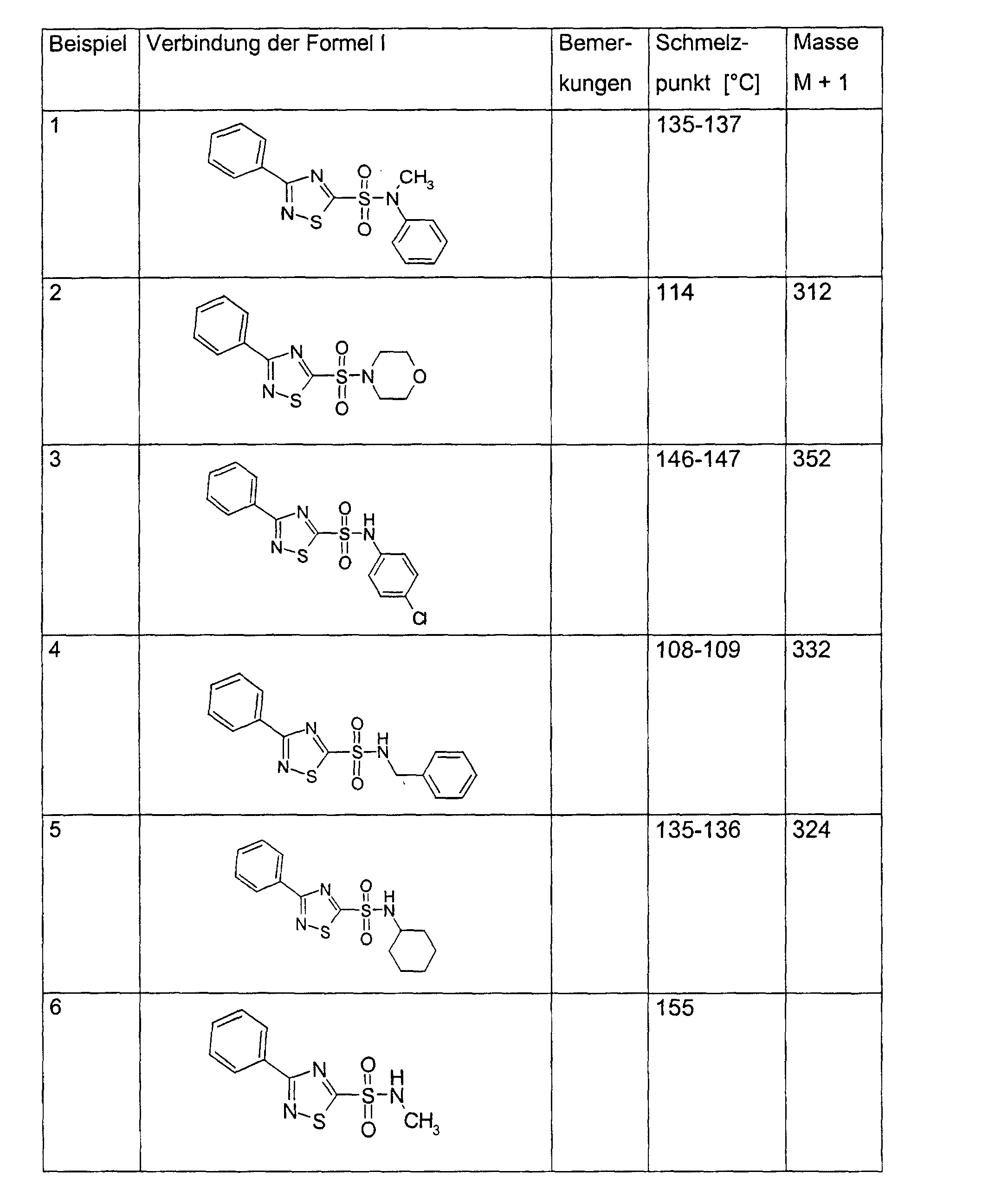

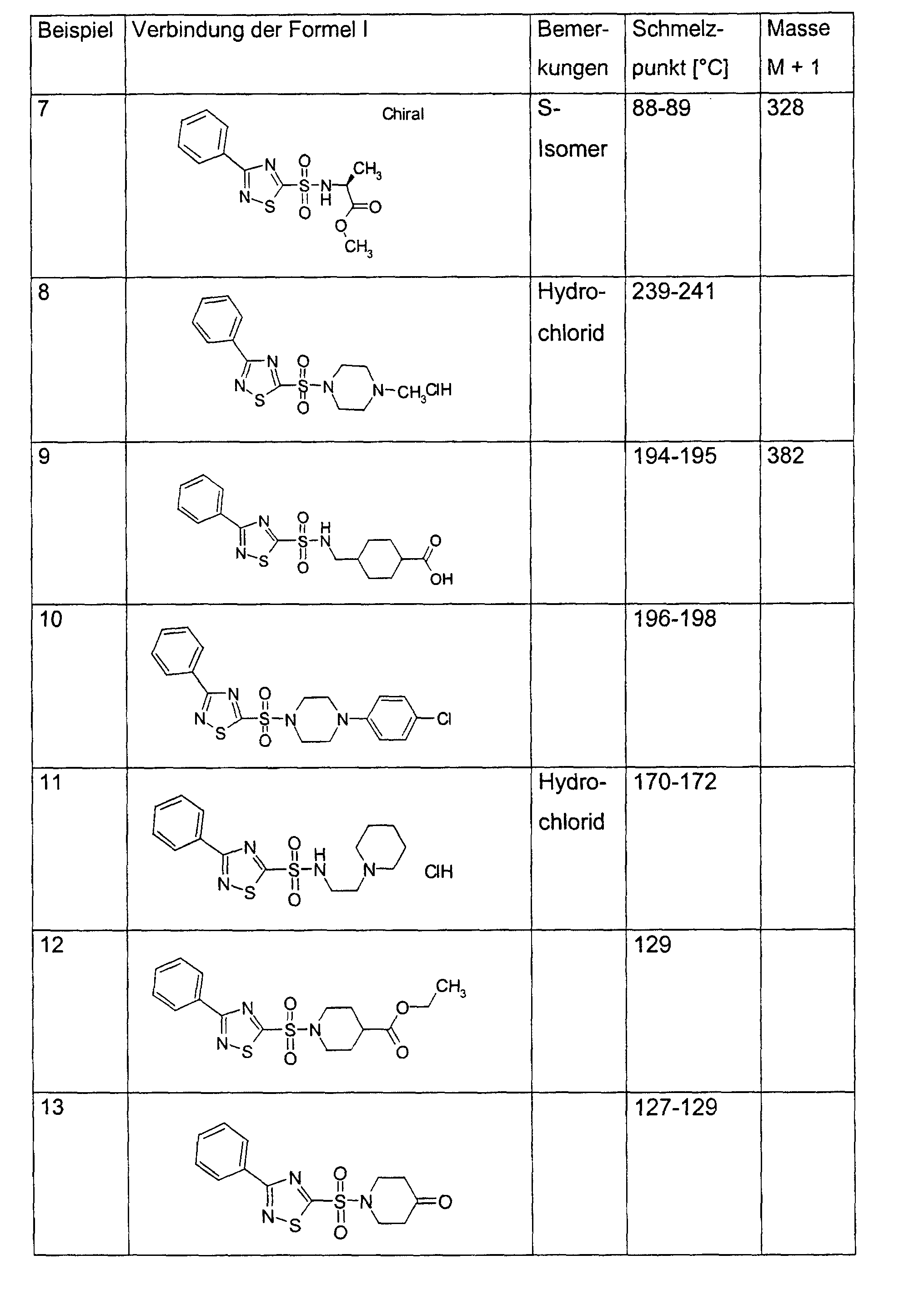

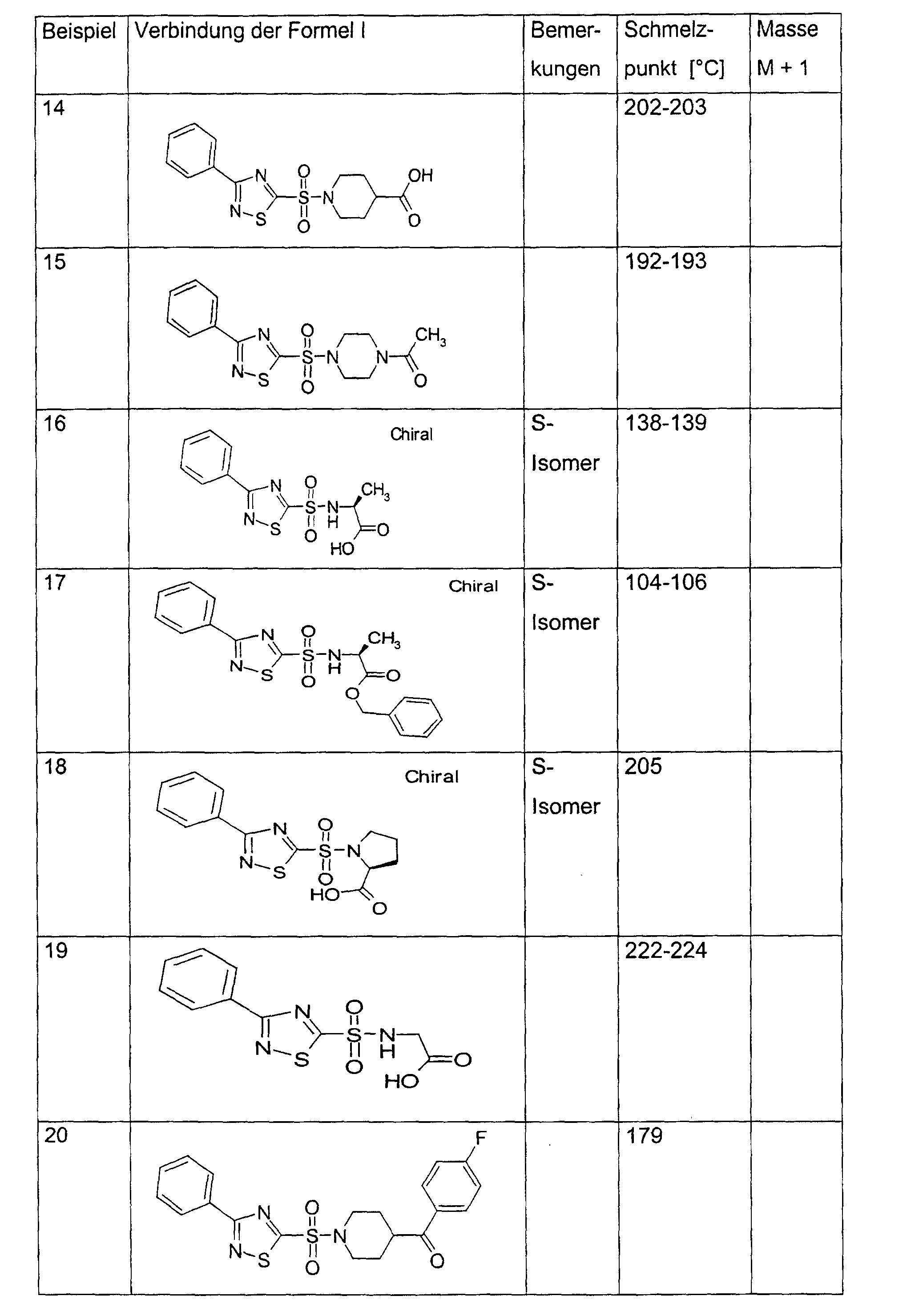

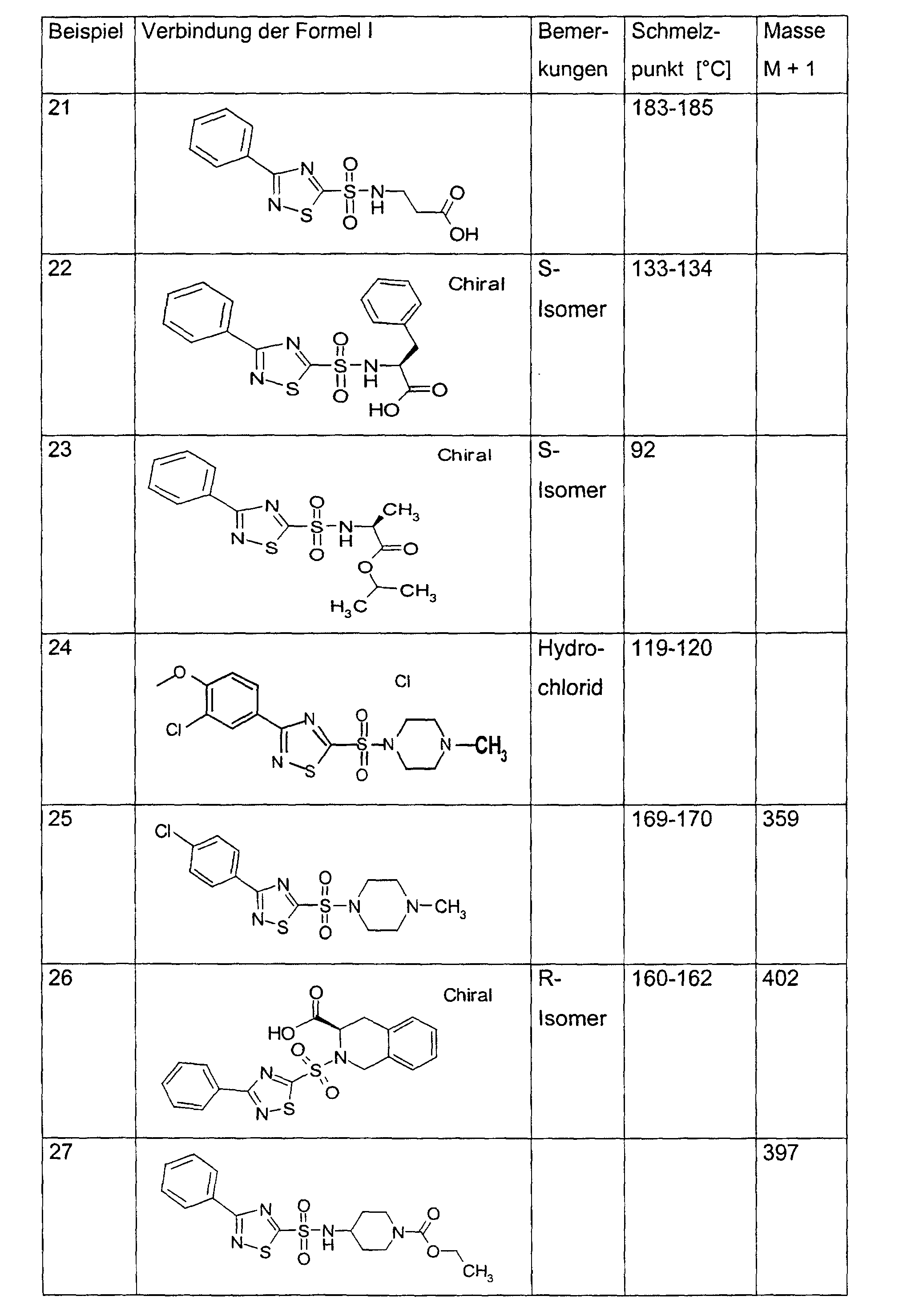

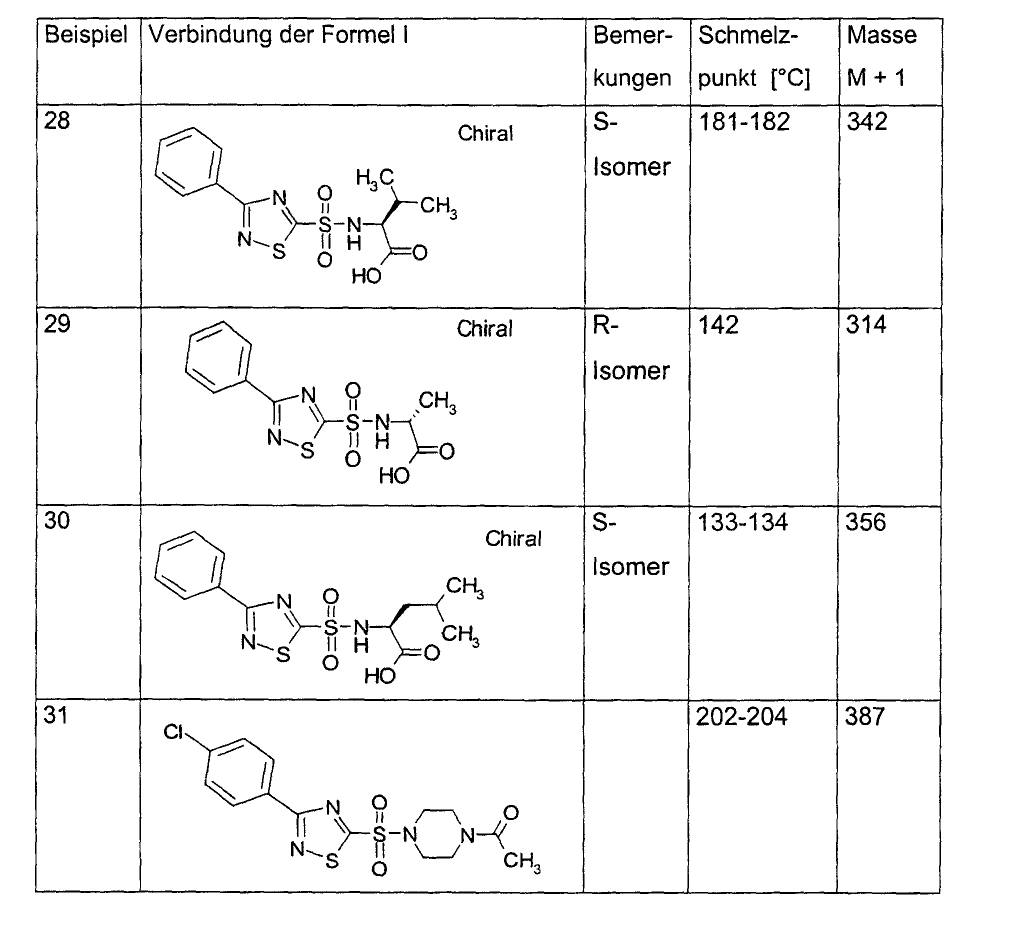

### Pharmakologische Beispiele

Experimentell konnten die Wirkungen der erfindungsgemäßen Verbindungen auf die IL1-Freisetzung an einer isolierten Blutzellfraktion (mononukleare Zellen) nachgewiesen werden.

### Beispiel:

Die Anreicherung der mononuklearen Zellen aus frisch gewonnenem menschlichen Zitratblut erfolgte nach bekannten Standardverfahren (s. Tiku et al, J. Immunol. 136/10 (1986) 3677):
10 ml frisch hergestelltes menschliches Zitratblut wurde vorsichtig mit 15 ml Lymphoprep^{(R)} (Molter GmbH, Heidelberg, FRG) unterschichtet, und dann bei 400 xg (Minifuge^{(R)}, Fa. Heraeus, Hanau, FRG) 40 min bei 20° C zentrifugiert. Die sich in der Phasengrenze als weißer Ring abzeichnende Zellfraktion wurde mit Hilfe einer Spritze herausgezogen, 1:1 (v/v) mit PM-16-Puffer (Fa. Serva, Heidelberg, FRG) verdünnt, und nochmals, wie oben, für 10 min zentrifugiert. Der Überstand wurde mit 10 ml RPMI 1640-Puffer (Gibco, Berlin, FRG), dem vorher 300 mg/l L-Glutamin, 25 mmol/l HEPES, 0,1 g/ml Streptomyzin und 0,1 g/ml Penizillin zugesetzt worden waren, aufgenommen. Mit Hilfe eines Zellzählers (Typ IT, Fa. Coulter Diagnostics, Krefeld, FRG) wurde die Zellsuspension, die aus etwa 90% Lymphozyten und 10% Monozyten besteht, auf etwa 5 Millionen Zellen/ml eingestellt. Die Zellviabilität wurde vor und nach den Inhibierungsexperimenten mit Hilfe der bekannten Laktatdehydrogenase-Methode überprüft. Eine Änderung der Viabilität wurde dabei nicht festgestellt.
Die Synthese und Freisetzung von zellulärem IL-1β wurde induziert, indem zu 0,48 ml der oben beschriebenen Zellfraktion eine Lösung von 500 mg Lipopolysaccharid (Salmonella abortus equi, Sigma GmbH, Deisenhofen, FRG) in 0,01 ml Dimethylsufoxid/Wasser (1:10, v/v) gegeben wurde. Gleichzeitig wurde die Zellfraktion mit einer Lösung der Prüfsubstanz in 0,01 ml versetzt, und für 20 Stunden bei 37° C in einem handelsüblichen Inkubator belassen. Nach dem Kühlen der Proben auf 0° C wurde 1 Minute in einer Tischzentrifuge zentrifugiert, und jeweils 0,025 ml - Aliquote des Überstandes mit Hilfe eines kommerziell angebotenen "Sandwich" Enzym-Immuno-Testkits (Fa. Biosource, Ratingen, FRG) nach Herstellervorschrift spezifisch auf ihren IL-1β-Gehalt untersucht. Die Kontrollwerte wurden ohne Zusatz von Prüfpräparat bestimmt und auf 100% gesetzt. Insbesondere wurde durch entsprechende Vergleichsmessungen ein etwaiger Einfluß von Dimethylsulfoxid auf den IL-1 β-Spiegel ausgeschlossen.

Die dosisabhängige IL-1β-hemmende Wirkung einer Verbindung der Formel I wurde aufgezeigt, indem deren Wirkstärke (vollständige Hemmung der IL-1β-Freisetzung entspricht 100% Hemmung) bei sieben verschiedenen Konzentrationen bestimmt wurde. Der Konzentrationsbereich wurde dabei so gewählt, daß er die Bereiche von 0% Hemmung (also ohne Prüfsubstanzzusatz) bis mindestens 80% Hemmung umfaßte. Aus der graphisch oder rechnerisch ermittelten Dosis-Wirkungsbeziehung wurde die Prüfsubstanzkonzentration extrapoliert, die zu 50% Hemmung der IL-1β-Freisetzung führte.
Dieser IC₅₀-Wert wurde für die entsprechenden erfindungsgemäßen Verbindungen der Formel I in Tabelle 2 als micromol/Liter dargestellt.

**Tabelle 2**

| Beispiel Nummer | IC₅₀ [µM] | Beispiel Nummer | IC₅₀ [µM] |
|---|---|---|---|
| 1 | 11 | 16 | 10 |
| 2 | 3 | 17 | 10 |
| 3 | 34 | 18 | 8 |
| 4 | 15 | 19 | 40 |
| 5 | 6 | 20 | 12 |
| 7 | 2 | 21 | 6 |
| 8 | 4 | 22 | 20 |
| 9 | 2 | 23 | 6 |
| 10 | 10 | 24 | 20 |
| 12 | 2 | 25 | 2 |
| 13 | 2 | | |
| 14 | 2 | | |
| 15 | 2 | | |

## Patentansprüche

1. Die Verbindung der Formel I 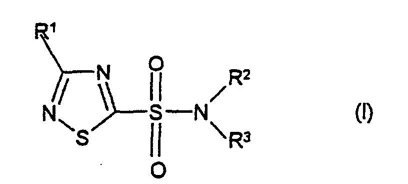 und/oder eine stereoisomere Form der Verbindung der Formel I und/oder ein physiologisch verträgliches Salz der Verbindung der Formel I, wobei
R¹ für
1. Phenyl,
2. Phenyl, welches ein- bis dreifach substituiert ist durch
2.1. -(C₁-C₆)-Alkyl, worin Alkyl gerade, cyclisch oder verzweigt ist,
2.2. -OH,
2.3. -O-C(O)-(C₁-C₆)-Alkyl-,
2.4. -O-(C₁-C₆)-Alkyl,
2.5. Methylendioxo,
2.6. Halogen,
2.7. -CF₃,
2.8. -CN,
2.9. -NO₂,
2.10. -C(O)-OH,
2.11. -C(O)-O-(C₁-C₆)-Alkyl oder
2.12. R³-(R⁴ )N-, worin R³ und R⁴ gleich oder verschieden sind und Wasserstoffatom oder (C₁-C₆)-Alkyl- bedeuten, steht, oder
3. einen Heteroaromaten aus der nachfolgenden Gruppe 3.1. bis 3.15., der unsubstituiert oder wie unter 2.1 bis 2.12. beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12 Benzothiophen,
3.13. Benzimidazol,
3.14. Benzoxazoloder
3.15. Benzothiazol, steht,
R³ für
1. Wasserstoffatom oder
2. -(C₁-C₆)-Alkyl steht,
R² für
1. -(C₁-C₇)-Alkyl, worin Alkyl verzweigt oder unverzweigt ist und ein- oder zweifach wie oben unter 2.1 bis 2.12. beschrieben substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie oben unter 2.1 bis 2.12. beschrieben substituiert ist,
2. -(C₂-C₁₀)-Alkenyl, worin Alkenyl gerade oder verzweigt ist,
3. -(C₃-C₇)-Cycloalkyl, worin Cycloalkyl unsubstituiert ist oder ein- oder zweifach wie oben unter 2.1 bis 2.12. beschrieben substituiert ist,
4. Phenyl, worin Phenyl unsubstituiert oder ein- oder zweifach wie oben unter 2.1. bis 2.12. beschrieben substituiert ist,
5. -(CH₂)ₘ-Phenyl, worin Phenyl unsubstituiert oder ein- oder zweifach wie oben unter 2.1. bis 2.12. beschrieben substituiert ist, m die ganze Zahl 1, 2, 3, 4, 5 oder 6 darstellt, oder ein Wasserstoffatom des -(CH₂)ₘ-Restes durch -OH ersetzt ist,
6. -(CH₂)ₙ-Heteroaryl, worin Heteroaryl wie unter 3.1. bis 3.15. definiert ist und unsubstituiert oder wie oben unter 2.1 bis 2.12. beschrieben substituiert ist und n die ganze Zahl Null, 1, 2, 3, 4, 5 oder 6 darstellt,
7. -(C₂-C₆)-Alkyl-O-R⁶,
worin R⁶ Wasserstoffatom, Benzyl, Allyl oder (C₁-C₆)-Alkyl darstellt und worin der Alkylrest gerade oder verzweigt ist,
8. -(C₂-C₆)-Alkyl-C(O)-OR⁶, worin R⁶ wie oben definiert ist,
9. -C(R⁸,R⁷)-C(O)-X, worin R⁸ die gleiche Bedeutung wie oben R³ hat, R⁷ den Rest einer α-Aminosäure der Formel II bedeutet 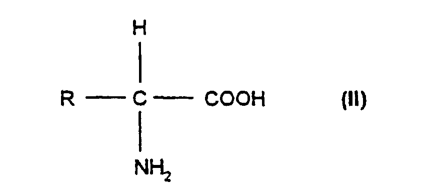 worin R sich von einer natürlichen oder unnatürlichen Aminosäure ableitet, oder R⁸ und R⁷ zusammen mit dem Kohlenstoffatom an dem sie gebunden sind einen 4- bis 7-gliedrigen Ring bilden, und X für -OH, NHR⁶ oder -OR⁶ steht, worin R⁶ wie oben definiert ist, steht oder
10. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
10.1 (C₁-C₂)-Alkyl,
10.2 Phenyl,
10.3 - OH,
10.4 = O,
10.5 -C(O)-O-(C₁-C₆)-Alkyl,
10.6 -C(O)-Phenyl, worin Phenyl unsubstituiert oder wie oben unter 2.1 bis 2.12 beschrieben substituiert ist,
10.7 -C(O)-(C₁-C₆)-Alkyl,oder
10.8 -C-(O)-OR³ , worin R³ Wasserstoffatom oder (C₁-C₆)-Alkyl darstellt, und/oder
10.9 zwei Kohlenstoffatome im Ring Teil eines zusätzlichen Phenylrestes bilden, der unsubstituiert oder wie oben unter 2.1 bis 2.12 beschrieben substituiert ist oder
worin das Wasserstoffatom in dem gegebenenfalls vorhandenen -NH- im Ring unsubstituiert ist oder durch
a) (C₁-C₃)-Alkyl,
b) -C(O)-R³, worin R³ wie oben definiert ist,
c) -(CH₂)ₒ-Phenyl, worin Phenyl unsubstituiert oder ein- oder zweifach wie unter 2.1 bis 2.12. beschrieben substituiert ist und o die ganze Zahl Null, 1 oder 2 darstellt,
d) Benzoyl, das unsubstituiert oder wie oben unter 2.1 bis 2.12 beschrieben substituiert ist oder durch
e) -C(O)-O-(C₁-C₆)-Alkyl ersetzt ist,
wobei die Verbindung, worin R¹ für Phenyl, R² für Phenyl und R³ für Wasserstoffatom stehen, ausgeschlossen ist.

2. Verbindung der Formel I gemäß Anspruch 1, **dadurch gekennzeichnet, daß**
R¹ für
1. Phenyl oder
2. Phenyl, welches einfach substituiert ist durch
2.1 (C₁-C₆)-Alkyl, worin Alkyl gerade oder verzweigt ist,
2.2 Halogen ,
2.3 -O-(C₁-C₃)Alkyl oder
2.4 -N-R³-(R⁴), worin R³ und R⁴ gleich oder verschieden sind und Wasserstoffatom oder (C₁-C₆)-Alkyl- bedeuten, steht
R³ für Wasserstoffatom oder Methyl steht,
R² für
1. (C₁-C₄)-Alkyl, worin Alkyl verzweigt oder unverzweigt ist und einfach wie unter 2.1 bis 2.12. beschrieben oder durch Phenyl substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie unter 2.1 bis 2.12. beschrieben substituiert ist,
2. Cyclohexyl,
3. -C(R⁸,R⁷)-C(O)-X, worin R⁸ Wasserstoffatom bedeutet, R⁷ den Rest einer α- Aminosäure der Formel II bedeutet, worin R sich von einer natürlichen oder unnatürlichen Aminosäure ableitet, oder R⁸ und R⁷ zusammen mit dem Kohlenstoffatom an dem sie gebunden sind einen 4- bis 7-gliedrigen Ring bilden, und X für -OH oder -OR⁶ steht, worin R⁶ Benzyl oder (C₁-C₃)-Alkyl bedeutet, steht oder
4. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
4.1 Phenyl, unsubstituiert oder substituiert durch Halogen,
4.2 -OH,
4.3 = O,
4.4 -C(O)-OH,
4.5 -C(O)-(C₁-C₂)-Alkyl
4.6 (C₁-C₃)-Alkyl oder
4.7 -(O)-OR³ substituiert ist und worin R³ Wasserstoffatom oder (C₁-C₆)-Alkyl darstellt,
oder worin das Wasserstoffatom in dem gegebenenfalls vorhandenen - NH- im Ring unsubstituiert ist oder durch
a) (C₁-C₃)-Alkyl,
b) -C(O)-R³, worin R³ Wasserstoffatom oder (C₁-C₆)-Alkyl darstellt,
c) Phenyl-(CH₂)ₒ-, worin Phenyl unsubstituiert oder ein- oder zweifach wie unter 2.1 bis 2.12. beschrieben substituiert ist und o die ganze Zahl Null, 1 oder 2 darstellt, oder
d) C(O)-O-(C₁-C₃)-Alkyl ersetzt ist, und/oder
4.8 zwei Kohlenstoffatome im Ring Teil eines zusätzlichen Phenylrestes bilden.

3. Verbindung der Formel I gemäß Anspruch 1 oder 2, **dadurch gekennzeichnet, daß**
R¹ für
1. Phenyl oder
2. Phenyl, welches einfach substituiert ist durch
2.1 (C₁-C₃)-Alkyl, worin Alkyl gerade oder verzweigt ist,
2.2 Chlor oder
2.3 -O-Methyl steht,
R³ für Wasserstoffatom oder Methyl steht,
R² für
1. (C₁-C₄)-Alkyl, worin Alkyl verzweigt oder unverzweigt ist und einfach durch Phenyl, C(O)-OH, Halogen, Piperidin oder Cyclohexyl substituiert ist, worin Piperidin oder Cyclohexyl unsubstituiert ist oder ein- oder zweifach durch Halogen oder C(O)-OH substituiert ist,
2. Cyclohexyl,
3. -C(R⁸,R⁷)-C(O)-X, worin R⁸ Wasserstoffatom bedeutet, R⁷ Methyl bedeutet und X für -OH oder -OR⁶ steht, worin R⁶ Benzyl oder (C₁-C₃)-Alkyl bedeutet, steht oder
4. R² und R³ zusammen mit dem Stickstoff an dem sie gebunden sind einen Morpholin-, Piperidin-, Piperazin- oder Tetrahydroisochinolin-3-carbonsäurerest bilden, und worin die Kohlenstoffatome im Ring unsubstituiert sind oder ein- oder zweifach substituiert sind durch
4.1 Phenyl, substituiert durch Chlor oder Fluor
4.2 Methyl,
4.3 = O,
4.4 -C(O)-OH oder
4.5 -C(O)-Methyl,
oder worin das Wasserstoffatom in dem gegebenenfalls vorhandenen - NH- im Ring unsubstituiert ist oder durch
a) Methyl,
b) -C(O)-Methyl,
c) Phenyl; substituiert durch Chlor, oder
d) C(O)-O-Ethyl ersetzt ist.

4. Verfahren zur Herstellung der Verbindung der Formel I gemäß einem oder mehreren der Ansprüche 1 bis 3, **dadurch gekennzeichnet, daß** man
a) ein Amin der Formel III 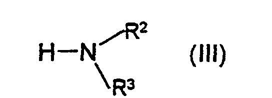 worin R² und R³ wie in Formel I definiert sind, mit einem Sulfonsäurederivat der Formel IV, 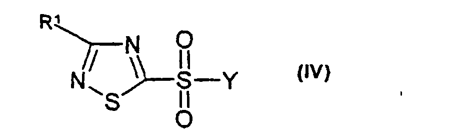 worin R¹ wie in Formel I definiert ist und Y ein Halogenatom, Imidazolyl, oder -OR¹⁰ bedeutet, worin R¹⁰ Wasserstoffatom, (C₁-C₆)-Alkyl, Phenyl oder Benzyl darstellt,
in Gegenwart einer Base oder gegebenenfalls eines wasserentziehenden Mittels zu einer Verbindung der Formel I umsetzt, oder
b) 5-Mercapto-thiadiazole der Formel V, 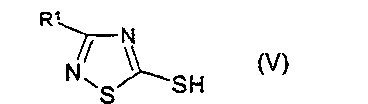 wobei R¹ die oben genannte Bedeutung hat,
in Gegenwart von Natriumhypochlorid oder Chlor und einem Amin der Formel III in ein Sulfenamine der Formel VI , 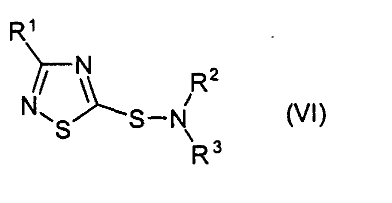 wobei R¹, R² und R³ die obengenannte Bedeutung haben, überführt und die Verbindung der Formel VI in Anwesenheit eines Oxydationsmittels wie Persäuren, insbesondere Peressigsäure, m-Chlorperbenzoesäure oder Wasserstoffperoxyd oder Kaliumpermanganat in die Verbindung Formel I umwandelt, oder
c) eine nach den Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a) oder b) hergestellte Verbindung der Formel I entweder in freier Form isoliert oder im Falle des Vorliegens von sauren oder basischen Gruppen in physiologisch verträgliche Salze umwandelt

5. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

6. Verwendung von mindestens einer Verbindung der Formel I 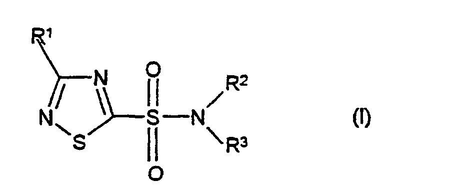 und/oder eine stereoisomere Form der Verbindung der Formel I und/oder ein physiologisch verträgliches Salz der Verbindung der Formel I, wobei
R¹ für
1. Phenyl,
2. Phenyl, welches ein- bis dreifach substituiert ist durch
2.1. -(C₁-C₆)-Alkyl, worin Alkyl gerade, cyclisch oder verzweigt ist,
2.2. -OH,
2.3. -O-C(O)-(C₁-C₆)-Alkyl-,
2.4. -O-(C₁-C₆)-Alkyl,
2.5. Methylendioxo,
2.6. Halogen,
2.7. -CF₃,
2.8. -CN,
2.9. -NO₂,
2.10. -C(O)-OH,
2.11. -C(O)-O-(C₁-C₆)-Alkyl oder
2.12. R³-(R⁴ )N-, worin R³ und R⁴ gleich oder verschieden sind und Wasserstoffatom oder (C₁-C₆)-Alkyl- bedeuten, steht, oder
3. einen Heteroaromaten aus der nachfolgenden Gruppe 3.1. bis 3.15., der unsubstituiert oder wie unter 2.1 bis 2.12. beschrieben substituiert ist,
3.1. Pyrrol,
3.2. Pyrazol,
3.3. Imidazol,
3.4. Triazol,
3.5. Thiophen,
3.6. Thiazol,
3.7. Oxazol,
3.8. Isoxazol,
3.9. Pyridin,
3.10. Pyrimidin,
3.11. Indol,
3.12 Benzothiophen,
3.13. Benzimidazol,
3.14. Benzoxazol oder
3.15. Benzothiazol, steht,
R³ für
1. Wasserstoffatom oder
2. -(C₁-C₆)-Alkyl steht,
R² für
1. -(C₁-C₇)-Alkyl, worin Alkyl verzweigt oder unverzweigt ist und unsubstituiert ist oder ein- oder zweifach wie oben unter 2.1 bis 2.12. beschrieben substituiert ist oder durch einen Heteroalkylring oder durch einen (C₃-C₇)-Cycloalkyl substituiert ist, worin der Heteroalkylring oder das (C₃-C₇)-Cycloalkyl unsubstituiert ist oder ein- oder zweifach wie oben unter 2.1 bis 2.12. beschrieben substituiert ist,
2. -(C₂-C₁₀)-Alkenyl, worin Alkenyl gerade oder verzweigt ist,
3. -(C₃-C₇)-Cycloalkyl, worin Cycloalkyl unsubstituiert ist oder ein- oder zweifach wie oben unter 2.1 bis 2.12. beschrieben substituiert ist,
4. Phenyl, worin Phenyl unsubstituiert oder ein- oder zweifach wie oben unter 2.1. bis 2.12. beschrieben substituiert ist,
5. -(CH₂)ₘ-Phenyl, worin Phenyl unsubstituiert oder ein- oder zweifach wie oben unter 2.1. bis 2.12. beschrieben substituiert ist, m die ganze Zahl 1, 2, 3, 4, 5 oder 6 darstellt, oder ein Wasserstoffatom des -(CH₂)ₘ-Restes durch -OH ersetzt ist,
6. -(CH₂)ₙ-Heteroaryl, worin Heteroaryl wie unter 3.1. bis 3.15. definiert ist und unsubstituiert oder wie oben unter 2.1 bis 2.12. beschrieben substituiert ist und n die ganze Zahl Null, 1, 2, 3, 4, 5 oder 6 darstellt,
7. -(C₂-C₆)-Alkyl-O-R⁶,
worin R⁶ Wasserstoffatom, Benzyl, Allyl oder (C₁-C₆)-Alkyl darstellt und worin der Alkylrest gerade oder verzweigt ist,
8. -(C₂-C₆)-Alkyl-C(O)-OR⁶, worin R⁶ wie oben definiert ist,
9. -C(R⁸,R⁷)-C(O)-X, worin R⁸ die gleiche Bedeutung wie oben R³ hat, R⁷ den Rest einer α-Aminosäure der Formel II bedeutet 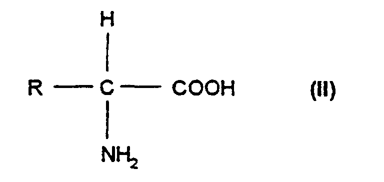 worin R sich von einer natürlichen oder unnatürlichen Aminosäure ableitet, oder R⁸ und R⁷ zusammen mit dem Kohlenstoffatom an dem sie gebunden sind einen 4- bis 7-gliedrigen Ring bilden, und X für -OH, NHR⁶ oder -OR⁶ steht, worin R⁶ wie oben definiert ist, steht oder
10. R² und R³ zusammen mit dem Stickstoff an dem sie gebunden sind einen 4- bis 7-gliedrigen Ring bilden, worin gegebenenfalls eines der Kohlenstoffatome durch -O-, -S- oder -NH- ersetzt ist, und worin ein, zwei oder drei der Kohlenstoffatome im Ring unsubstituiert sind oder ein- oder zweifach substituiert sind durch
10.1 (C₁-C₂)-Alkyl,
10.2 Phenyl,
10.3 -OH,
10.4 = O ,
10.5 -C(O)-O-(C₁-C₆)-Alkyl,
10.6 -C(O)-Phenyl, worin Phenyl unsubstituiert oder wie oben unter 2.1 bis 2.12 beschrieben substituiert ist,
10.7 -C(O)-(C₁-C₆)-Alkyl,oder
10.8 -C-(O)-OR³, worin R³ Wasserstoffatom oder (C₁-C₆)-Alkyl darstellt, und/oder
10.9 zwei Kohlenstoffatome im Ring Teil eines zusätzlichen Phenylrestes bilden, der unsubstituiert oder wie oben unter 2.1 bis 2.12 beschrieben substituiert ist oder
worin das Wasserstoffatom in dem gegebenenfalls vorhandenen -NH- im Ring unsubstituiert ist oder durch
a) (C₁-C₃)-Alkyl,
b) -C(O)-R³, worin R³ wie oben definiert ist,
c) -(CH₂)ₒ-Phenyl, worin Phenyl unsubstituiert oder ein- oder zweifach wie unter 2.1 bis 2.12. beschrieben substituiert ist und o die ganze Zahl Null, 1 oder 2 darstellt,
d) Benzoyl, das unsubstituiert oder wie oben unter 2.1 bis 2.12 beschrieben substituiert ist oder durch
e) -C(O)-O-(C₁-C₆)-Alkyl ersetzt ist,
zur Herstellung von Arzneimitteln zur Prophylaxe und Therapie von Erkrankungen, die durch IL-1β gekennzeichnet sind oder solchen, an deren Entstehung IL-1β beteiligt ist, insbesondere solchen, an deren Verlauf erhöhte Konzentrationen von IL-1β beteiligt sind ist, wie Leukämie, septischer Schock, Hepatitis, HIV-Infektionen oder musculoskeletale Erkrankungen wie Muskelabbau, degenerative Gelenkerkrankungen wie Osteoarthrosen, Spondylosen, Knorpelschwund nach Gelenktrauma oder längerer Gelenksruhigstellung nach Meniskus- oder Patellaverietzungen oder Bänderrissen oder Erkrankungen des Bindegewebes wie Kollagenosen, Periodontalerkrankungen, Wundheilungsstörungen und insbesondere chronische Erkrankungen des Bewegungsapparates wie entzündliche, immunologisch oder stoffwechselbedingte akute und chronische Arthritiden, Arthropathien, rheumatoide Arthritis, Myalgien und Störungen des Knochenstoffwechsels.

7. Verfahren zur Herstellung eines Arzneimittels , **dadurch gekennzeichnet, daß** man mindestens eine Verbindung der Formel I gemäß einem oder mehreren der Ansprüche 1 bis 3 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. The compound of the formula I 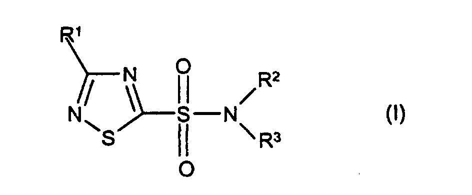 and/or a stereoisomeric form of the compound of the formula I and/or a physiologically tolerated salt of the compound of the formula I, in which
R¹ is
1. phenyl,
2. phenyl which is mono- to trisubstituted by
2.1. -(C₁-C₆)-alkyl, wherein alkyl is straight-chain, cyclic or branched,
2.2. -OH,
2.3. -O-C(O)-(C₁-C₆)-alkyl-,
2.4. -O-(C₁-C₆)-alkyl,
2.5. methylenedioxo,
2.6. halogen,
2.7. -CF₃,
2.8. -CN,
2.9. -NO₂,
2.10. -C(O)-OH,
2.11. -C(O)-O-(C₁-C₆)-alkyl or
2.12. R³-(R⁴)N-, wherein R³ and R⁴ are identical or different and are a hydrogen atom or (C₁-C₆)-alkyl, or
3. a heteroaromatic from the following group 3.1. to 3.15., which is unsubstituted or substituted as described under 2.1. to 2.12.,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12. benzothiophene,
3.13. benzimidazole,
3.14. benzoxazole or
3.15. benzothiazole,
R³ is
1. a hydrogen atom or
2. -(C₁-C₆)-alkyl,
R² is
1. -(C₁-C₇)-alkyl, wherein alkyl is branched or unbranched and is mono- or disubstituted as described above under 2.1. to 2.12. or is substituted by a heteroalkyl ring or by a (C₃-C₇)-cycloalkyl, wherein the heteroalkyl ring or the (C₃-C₇)-cycloalkyl is unsubstituted or is mono- or disubstituted as described above under 2.1. to 2.12.,
2. -(C₂-C₁₀)-alkenyl, wherein alkenyl is straight-chain or branched,
3. -(C₃-C₇)-cycloalkyl, wherein cycloalkyl is unsubstituted or is mono- or disubstituted as described above under 2.1. to 2.12.,
4. phenyl, wherein phenyl is unsubstituted or mono- or disubstituted as described above under 2.1. to 2.12.,
5. -(CH₂)ₘ-phenyl, wherein phenyl is unsubstituted or mono- or disubstituted as described above under 2.1. to 2.12. and m is the integer 1, 2, 3, 4, 5 or 6, or one hydrogen atom of the -(CH₂)ₘ radical is replaced by -OH,
6. -(CH₂)ₙ-heteroaryl, wherein heteroaryl is as defined under 3.1. to 3.15. and is unsubstituted or substituted as described above under 2.1. to 2.12. and n is the integer zero, 1, 2, 3, 4, 5 or 6,
7. -(C₂-C₆)-alkyl-O-R⁶,
wherein R⁶ is a hydrogen atom, benzyl, allyl or (C₁-C₆)-alkyl and wherein the alkyl radical is straight-chain or branched,
8. -(C₂-C₆)-alkyl-C(O)-OR⁶, wherein R⁶ is as defined above,
9. -C(R⁸,R⁷)-C(O)-X, wherein R⁸ has the same meaning as R³ above, R⁷ is the radical of an α-amino acid of the formula II 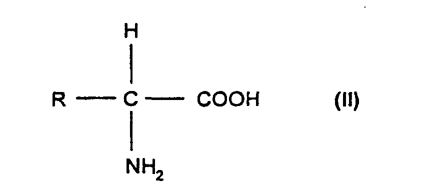 in which R is derived from a natural or non-natural amino acid, or R⁸ and R⁷, together with the carbon atom to which they are bonded, form a 4- to 7-membered ring, and X is -OH, NHR⁶ or -OR⁶, wherein R⁶ is as defined above, or
10. R² and R³, together with the nitrogen to which they are bonded, form a 4- to 7-membered ring, wherein one of the carbon atoms is optionally replaced by -O-, -S- or - NH-, and wherein one, two or three of the carbon atoms in the ring are unsubstituted or mono- or disubstituted by
10.1 (C₁-C₂)-alkyl,
10.2 phenyl,
10.3 - OH,
10.4 =O,
10.5 -C(O)-O-(C₁-C₆)-alkyl,
10.6 -C(O)-phenyl, wherein phenyl is unsubstituted or substituted as described above under 2.1. to 2.12.,
10.7 -C(O)-(C₁-C₆)-alkyl, or
10.8 -C-(O)-OR³ , wherein R³ is a hydrogen atom or (C₁-C₆)-alkyl, and/or
10.9 two carbon atoms in the ring form part of an additional phenyl radical, which is unsubstituted or substituted as described above under 2.1. to 2.12., or
wherein the hydrogen atom in the -NH- optionally present in the ring is unsubstituted or is replaced by
a) (C₁-C₃)-alkyl,
b) -C(O)-R³, wherein R³ is as defined above,
c) -(CH₂)ₒ-phenyl, wherein phenyl is unsubstituted or mono- or disubstituted as described under 2.1. to 2.12. and o is the integer zero, 1 or 2,
d) benzoyl, which is unsubstituted or substituted as described above under 2.1. to 2.12., or by
e) -C(O)-O-(C₁-C₆)-alkyl,
the compound in which R¹ is phenyl, R² is phenyl and R³ is a hydrogen atom being excluded.

2. A compound of the formula I as claimed in claim 1, wherein
R¹ is
1. phenyl or
2. phenyl which is monosubstituted by
2.1 (C₁-C₆)-alkyl, wherein alkyl is straight-chain or branched,
2.2 halogen,
2.3 -O-(C₁-C₃)alkyl or
2.4 -N-R³-(R⁴), wherein R³ and R⁴ are identical or different and are a hydrogen atom or (C₁-C₆)-alkyl,
R³ is a hydrogen atom or methyl,
R² is
1. -(C₁-C₄)-alkyl, wherein alkyl is branched or unbranched and is monosubstituted as described under 2.1. to 2.12. or by phenyl or is substituted by a heteroalkyl ring or by a (C₃-C₇)-cycloalkyl, wherein the heteroalkyl ring or the (C₃-C₇)-cycloalkyl is unsubstituted or is mono- or disubstituted as described under 2.1. to 2.12.,
2. cyclohexyl,
3. -C(R⁸,R⁷)-C(O)-X, wherein R⁸ is a hydrogen atom, R⁷ is the radical of an α-amino acid of the formula II, in which R is derived from a natural or non-natural amino acid, or R⁸ and R⁷, together with the carbon atom to which they are bonded, form a 4- to 7-membered ring, and X is -OH or -OR⁶, wherein R⁶ is benzyl or (C₁-C₃)-alkyl, or
4. R² and R³, together with the nitrogen to which they are bonded, form a 4- to 7-membered ring, wherein one of the carbon atoms is optionally replaced by -O-, -S- or - NH-, and wherein one, two or three of the carbon atoms in the ring are unsubstituted or are mono- or disubstituted by
4.1 phenyl, which is unsubstituted or substituted by halogen,
4.2 -OH,
4.3 = O,
4.4 -C(O)-OH,
4.5 -C(O)-(C₁-C₂)-alkyl,
4.6 (C₁-C₃)-alkyl or
4.7 -(O)-OR³ and wherein R³ is a hydrogen atom or (C₁-C₆)-alkyl,
or wherein the hydrogen atom in the -NH- optionally present in the ring is unsubstituted or is replaced by
a) (C₁-C₃)-alkyl,
b) -C(O)-R³, wherein R³ is a hydrogen atom or (C₁-C₆)-alkyl,
c) phenyl-(CH₂)ₒ-, wherein phenyl is unsubstituted or mono- or disubstituted as described under 2.1. to 2.12. - and o is the integer zero, 1 or 2, or
d) C(O)-O-(C₁-C₃)-alkyl and/or
4.8 two carbon atoms in the ring form part of an additional phenyl radical.

3. A compound of the formula I as claimed in claim 1 or 2, wherein
R¹ is
1. phenyl or
2. phenyl which is monosubstituted by
2.1 (C₁-C₃)-alkyl, wherein alkyl is straight-chain or branched,
2.2 chlorine or
2.3 -O-methyl,
R³ is a hydrogen atom or methyl,
R² is
1. (C₁-C₄)-alkyl, wherein alkyl is branched or unbranched and is monosubstituted by phenyl, C(O)-OH, halogen, piperidine or cyclohexyl, wherein piperidine or cyclohexyl is unsubstituted or is mono- or disubstituted by halogen or C(O)-OH,
2. cyclohexyl,
3. -C(R⁸,R⁷)-C(O)-X, wherein R⁸ is a hydrogen atom, R⁷ is methyl and X is -OH or -OR⁶, wherein R⁶ is benzyl or (C₁-C₃)-alkyl, or
4. R² and R³, together with the nitrogen to which they are bonded, form a morpholine-, piperidine-, piperazine- or tetrahydroisoquinoline-3-carboxylic acid radical, and wherein the carbon atoms in the ring are unsubstituted or are mono- or disubstituted by
4.1 phenyl, which is substituted by chlorine or fluorine,
4.2 methyl,
4.3 = O,
4.4 -C(O)-OH or
4.5 -C(O)-methyl,
or wherein the hydrogen atom in the -NH- optionally present in the ring is unsubstituted or is replaced by
a) methyl,
b) -C(O)-methyl,
c) - phenyl, which is substituted by chlorine, or
d) C(O)-O-ethyl.

4. A process for the preparation of a compound of the formula I as claimed in one or more of claims 1 to 3, which comprises
a) reacting an amine of the formula III 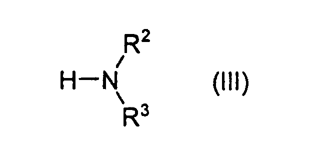 wherein R² and R³ are as defined in formula I, with a sulfonic acid derivative of the formula IV 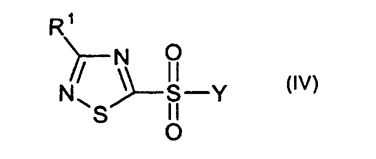 wherein R¹ is as defined in formula I and Y is a halogen atom, imidazolyl or -OR¹⁰, wherein R¹⁰ is a hydrogen atom, (C₁-C₆)-alkyl, phenyl or benzyl,
in the presence of a base or optionally a dehydrating agent, to give a compound of the formula I, or
b) converting a 5-mercapto-thiadiazole of the formula V 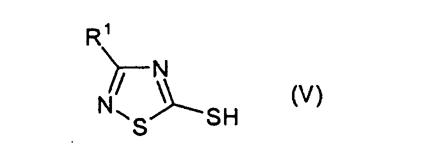 in which R¹ has the abovementioned meaning,
in the presence of sodium hypochlorite or chlorine and an amine of the formula III into a sulfenamine of the formula VI 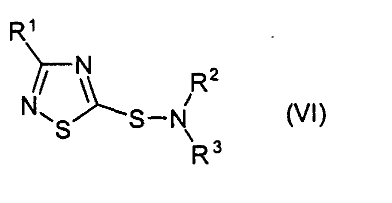 in which R¹, R² and R³ have the abovementioned meaning, and converting the compound of the formula VI, in the presence of an oxidizing agent, such as per acids, in particular peracetic acid, or m-chtoroperbenzoic acid, or hydrogen peroxide or potassium permanganate, into the compound of the formula I, or
c) separating a compound of the formula I prepared by processes a) or b) which, because of its chemical structure, occurs in enantiomeric forms, into the pure enantiomers by salt formation with enantiomerically pure acids or bases, chromatography over chiral stationary phases or derivatization by means of chiral enantiomerically pure compounds, such as amino acids, separation of the diastereomers thus obtained and elimination of the chiral auxiliary groups, or
d) either isolating the compound of the formula I prepared by processes a) or b) in the free form or, in the case where acid or basic groups are present, converting it into physiologically tolerated salts.

5. A pharmaceutical which has an active content of at least one compound of the formula I as claimed in one or more of claims 1 to 3 together with a pharmaceutically suitable and physiologically tolerated excipient, additive and/or other active compounds and auxiliaries.

6. The use of at least one compound of the formula I 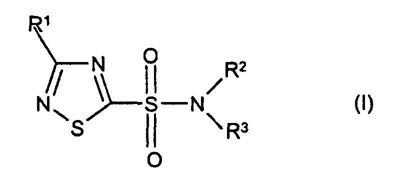 and/or a stereoisomeric form of the compound of the formula I and/or a physiologically tolerated salt of the compound of the formula I, in which
R¹ is
1. phenyl,
2. phenyl which is mono- to trisubstituted by
2.1. -(C₁-C₆)-alkyl, wherein alkyl is straight-chain, cyclic or branched,
2.2. -OH,
2.3. -O-C(O)-(C₁-C₆)-alkyl-,
2.4. -O-(C₁-C₆)-alkyl,
2.5. methylenedioxo,
2.6. halogen,
2.7. -CF₃,
2.8. -CN,
2.9. -NO₂,
2.10. -C(O)-OH,
2.11. -C(O)-O-(C₁-C₆)-alkyl or
2.12. R³-(R⁴)N-, wherein R³ and R⁴ are identical or different and are a hydrogen atom or (C₁-C₆)-alkyl, or
3. a heteroaromatic from the following group 3.1. to 3.15., which is unsubstituted or substituted as described under 2.1. to 2.12.,
3.1. pyrrole,
3.2. pyrazole,
3.3. imidazole,
3.4. triazole,
3.5. thiophene,
3.6. thiazole,
3.7. oxazole,
3.8. isoxazole,
3.9. pyridine,
3.10. pyrimidine,
3.11. indole,
3.12. benzothiophene,
3.13. benzimidazole,
3.14. benzoxazole or
3.15. benzothiazole,
R³ is
1. a hydrogen atom or
2. -(C₁-C₆)-alkyl,
R² is
1. -(C₁-C₇)-alkyl, wherein alkyl is branched or unbranched and is unsubstituted or is mono- or disubstituted as described above under 2.1. to 2.12. or is substituted by a heteroalkyl ring or by a (C₃-C₇)-cycloalkyl, wherein the heteroalkyl ring or the (C₃-C₇)-cycloalkyl is unsubstituted or is mono- or disubstituted as described above under 2.1. to 2.12.,
2. -(C₂-C₁₀)-alkenyl, wherein alkenyl is straight-chain or branched,
3. -(C₃-C₇)-cycloalkyl, wherein cycloalkyl is unsubstituted or is mono- or disubstituted as described above under 2.1. to 2.12.,
4. phenyl, wherein phenyl is unsubstituted or mono- or disubstituted as described above under 2.1. to 2.12.,
5 -(CH₂)ₘ-phenyl, wherein phenyl is unsubstituted or mono- or disubstituted as described above under 2.1. to 2.12. and m is the integer 1, 2, 3, 4, 5 or 6, or one hydrogen atom of the -(CH₂)ₘ radical is replaced by -OH,
6. -(CH₂)ₙ-heteroaryl, wherein heteroaryl is as defined under 3.1. to 3.15. and is unsubstituted or substituted as described above under 2.1. to 2.12. and n is the integer zero, 1, 2, 3, 4, 5 or 6,
7. -(C₂-C₆)-alkyl-O-R⁶, wherein R⁶ is a hydrogen atom, benzyl, allyl or (C₁-C₆)-alkyl and wherein the alkyl radical is straight-chain or branched,
8. -(C₂-C₆)-alkyl-C(O)-OR⁶, wherein R⁶ is as defined above,
9. -C(R⁸,R⁷)-C(O)-X, wherein R⁸ has the same meaning as R³ above, R⁷ is the radical of an α-amino acid of the formula II 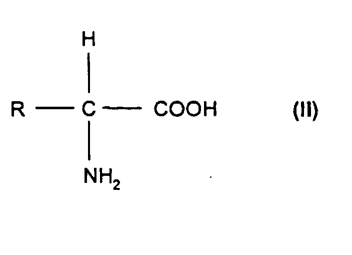 in which R is derived from a natural or non-natural amino acid, or R⁸ and R⁷, together with the carbon atom to which they are bonded, form a 4- to 7-membered ring, and X is -OH, NHR⁶ or -OR⁶, wherein R⁶ is as defined above, or
10. R² and R³, together with the nitrogen to which they are bonded, form a 4- to 7-membered ring, wherein one of the carbon atoms is optionally replaced by -O-, -S- or - NH-, and wherein one, two or three of the carbon atoms in the ring are unsubstituted or mono- or disubstituted by
10.1 (C₁-C₂)-alkyl,
10.2 phenyl,
10.3 -OH,
10.4 =O,
10.5 -C(O)-O-(C₁-C₆)-alkyl,
10.6 -C(O)-phenyl, wherein phenyl is unsubstituted or substituted as described above under 2.1. to 2.12.,
10.7 -C(O)-(C₁-C₆)-alkyl, or
10.8 -C-(O)-OR³ , wherein R³ is a hydrogen atom or (C₁-C₆)-alkyl, and/or
10.9 two carbon atoms in the ring form part of an additional phenyl radical, which is unsubstituted or substituted as described above under 2.1. to 2.12., or
wherein the hydrogen atom in the -NH- optionally present in the ring is unsubstituted or is replaced by
a) (C₁-C₃)-alkyl,
b) -C(O)-R³, wherein R³ is as defined above,
c) -(CH₂)ₒ-phenyl, wherein phenyl is unsubstituted or mono- or disubstituted as described under 2.1. to 2.12. and o is the integer zero, 1 or 2,
d) benzoyl, which is unsubstituted or substituted as described above under 2.1. to 2.12., or by
e) -C(O)-O-(C₁-C₆)-alkyl,
for the preparation of a pharmaceutical for the prophylaxis and treatment of diseases which are **characterized by** IL-1β, or those where IL-1β participates in their development, in particular those where increased concentrations of IL-1β participate in their course, such as leukemia, septic shock, hepatitis, HIV infections or musculoskeletal diseases, such as muscular degeneration, degenerative joint diseases, such as osteoarthrosis, spondylosis, chondrolysis following joint trauma or prolonged immobilization of joints following meniscus or patella injuries, or tom ligaments, or diseases of the connective tissue, such as collagenosis, periodontal diseases, wound-healing disturbances and, in particular, chronic diseases of the locomotor system, such as inflammatory or immunologically or metabolism-related acute and chronic arthritis, arthropathies, rheumatoid arthritis, myalgias and disturbances in bone metabolism.

7. A process for the preparation of a pharmaceutical, which comprises bringing at least one compound of the formula I as claimed in one or more of claims 1 to 3 into a suitable presentation form with a pharmaceutically suitable and physiologically tolerated excipient and optionally further suitable active compounds, additives or auxiliaries.

## Revendications

1. Composé de formule I 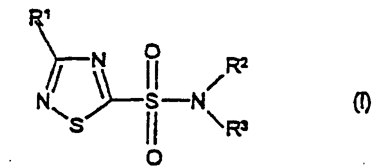 et/ou une forme stéréoisomère du composé de formule I et/ou un sel physiologiquement compatible du composé de formule I, où
R¹ représente
1. phényle,
2. phényle, qui est substitué une à trois fois par des substituants
2.1. -alkyle en C₁-C₆, où alkyle est linéaire, cyclique ou ramifié,
2.2. -OH,
2.3. -O-C(O)-alkyle en C₁-C₆,
2.4. -O-alkyle en C₁-C₆,
2.5. méthylènedioxo,
2.6. halogène,
2.7. -CF₃,
2.8. -CN,
2.9. -NO₂,
2.10. -C(O)-OH,
2.11. -C(O)-O-alkyle en C₁-C₆ ou
2.12. R³(R⁴)N-, où R³ et R⁴ sont identiques ou différents et représentent un atome d'hydrogène ou un groupe alkyle en C₁-C₆, ou
3. un hétéroatome pris dans les groupes suivants 3.1 à 3.15, qui est non substitué ou substitué comme décrit aux points 2.1 à 2.12,
3.1. pyrrole,
3.2. pyrazole,
3.3. imidazole,
3.4. triazole,
3.5. thiophène,
3.6. thiazole,
3.7. oxazole,
3.8. isoxazole,
3.9. pyridine,
3.10. pyrimidine,
3.11. indole,
3.12. benzothiophène,
3.13. benzimidazole,
3.14. benzoxazole ou
3.15. benzothiazole,
R³ représente
1. un atome d'hydrogène ou
2. un groupe alkyle en C₁-C₆,
R² représente
1. un groupe alkyle en C₁-C₇, où le groupe alkyle est linéaire ou ramifié et substitué une ou deux fois comme décrit ci-dessus aux points 2.1 à 2.12 ou par un cycle hétéroalkyle ou par un groupe cycloalkyle en C₃-C₇, le cycle hétéroalkyle ou le groupe cycloalkyle en C₃-C₇ est non substitué ou substitué une ou deux fois comme décrit ci-dessus aux points 2.1 à 2.12,
2. alcényle en C₂-C₁₀, alcényle est linéaire ou ramifié,
3. cycloalkyle en C₃-C₇, où cycloalkyle est non substitué ou substitué une ou deux fois comme décrit ci-dessus aux points 2.1 à 2.12,
4. phényle, où phényle est non substitué ou substitué une ou deux fois comme décrit ci-dessus aux points 2.1 à 2.12,
5. -(CH₂)ₘ-phényle, ou phényle est non substitué ou substitué une ou deux fois comme décrit ci-dessus aux points 2.1 à 2.12, m est un entier 1, 2, 3, 4, 5 ou 6, ou un atome d'hydrogène du reste - (CH₂)ₘ- est remplacé par -OH,
6. -(CH₂)ₙ-hétéroaryle, où le groupe hétéroaryle est défini comme aux points 3.1 à 3.15 et est non substitué ou substitué comme décrit ci-dessus aux point 2.1 à 2.12, et n est un entier zéro, 1, 2, 3, 4, 5 ou 6,
7. (alkyl en C₂-C₆) -O-R⁶, où R⁶ représente un atome d'hydrogène, un groupe benzyle, allyle ou alkyle en C₁-C₆ et où alkyle est linéaire ou ramifié,
8 . -(alkyl en C₂-C₆)-C(O)-OR⁶, où R⁶ est défini comme ci-dessus,
9. -C(R⁸,R⁷)-C(O)-X, où R⁸ possède la même signification que ci-dessus R³, R⁷ représente le reste d'un acide □-aminé de formule II 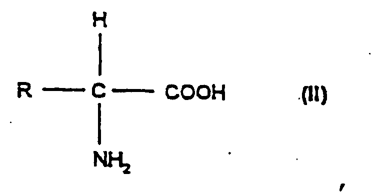 où R dérive d'un acide aminé naturel ou synthétique, ou R⁸ et R⁷, conjointement avec l'atome de carbone auquel ils sont liés, forment un cycle de 4 à 7 chaînons, et X représente -OH, NHR⁶ ou -OR⁶, où R⁶ est défini comme ci-dessus, ou
10.R² et R³, conjointement avec l'atome d'azote auquel ils sont liés, forment un cycle de 4 à 7 chaînons, où éventuellement un des atomes de carbone est remplacé par un -O-, -S- ou -NH-, et où un, deux ou trois des atomes de carbone du cycle sont non substitués ou substitués une ou deux fois par un substituant
10.1 alkyle en C₁-C₂,
10.2 phényle,
10.3 -OH,
10.4 =O,
10.5 -C(O)-O-alkyle en C₁-C₆,
10.6 -C(O)-phényle, où phényle est non substitué ou substitué comme décrit ci-dessus aux points 2.1 à 2.12,
10.7 -C(O)-alkyle en C₁-C₆ ou
10.8 -C-(O)-OR³, où R³ représente un atome d'hydrogène ou un groupe alkyle en C₁-C₆, et/ou
10.9 deux atomes de carbone dans le cycle font partie d'un reste phényle supplémentaire, qui est non substitué ou substitué comme décrit ci-dessus aux points 2.1 à 2.12 ou
où l'atome d'hydrogène dans le groupe -NH-éventuellement présent dans le cycle est non substitué ou substitué par un substituant
a) alkyle en C₁-C₃,
b) -C(O)-R³, où R³ est défini comme ci-dessus,
c) -(CH₂)ₒ-phényle, où phényle est non substitué ou substitué une ou deux fois comme décrit ci-dessus aux points 2.1 à 2.12 et o est un entier zéro, 1 ou 2,
d) benzoyle qui est non substitué ou substitué comme décrit ci-dessus aux points 2.1 à 2.12 ou
e) est remplacé par -C(O)-O-alkyle en C₁-C₆, le composé, où R¹ représente phényle, R² représente phényle et R³ représente un atome d'hydrogène, étant exclu.

2. Composé de formule I selon la revendication 1, **caractérisé en ce que**
R¹ représente
1. phényle ou
2. phényle, qui est substitué une fois par un substituant
2.1 alkyle en C₁-C₆, où alkyle est linéaire ou ramifié,
2.2 halogène,
2.3 -O-alkyle en C₁-C₃ ou
2.4 -N-R³-(R⁴), où R³ et R⁴ sont identiques ou différents et représentent un atome d'hydrogène ou un groupe alkyle en C₁-C₆,
R³ représente un atome d'hydrogène ou un groupe méthyle,
R² représente
1. un groupe alkyle en C₁-C₄, où alkyle est linéaire ou ramifié et substitué une fois comme décrit ci-dessus aux points 2.1 à 2.12, ou est substitué par un substituant phényle ou par un cycle hétéroalkyle ou par un groupe cycloalkyle en C₃-C₇, où le cycle hétéroalkyle ou le groupe cycloalkyle en C₃-C₇ est non substitué ou un ou deux fois substitué comme décrit aux points 2.1 à 2.12,
2. cyclohexyle,
3. -C (R⁸,R⁷)-C(O)-X, où R⁸ représente un atome d'hydrogène, R⁷ représente le reste d'un acide □-aminé de formule II, où R dérive d'un acide aminé naturel ou synthétique, ou R⁸ et R⁷, conjointement avec l'atome de carbone auquel ils sont liés, forment un cycle de 4 à 7 chaînons, et X représente un groupe -OH ou -OR⁶, où R⁶ représente un groupe benzyle ou alkyle en C₁-C₃, ou
4. R² et R³ conjointement avec l'atome d'azote auquel ils sont liés forment un cycle de 4 à 7 chaînons, où éventuellement l'un des atomes de carbone est remplacé par -O-, -S- ou -NH-, et où un, deux ou trois des atomes de carbone du cycle sont non substitués ou substitués une ou deux fois par un substituant
4.1 phényle, qui est non substitué ou substitué par un atome d'halogène,
4.2 -OH,
4.3 =O,
4.4 -C(O)-OH,
4.5 -C(O)-alkyle en C₁-C₂,
4.6 alkyle en C₁-C₃ ou
4.7 -(O)-OR³ et où R³ représente un atome d'hydrogène ou un groupe alkyle en C₁-C₆, ou dans lequel l'atome d'hydrogène dans le groupe -NH- éventuellement présent dans le cycle est non substitué ou substitué par un substituant
a) alkyle en C₁-C₃,
b) -C(O)-R³, où R³ représente un atome d'hydrogène ou un groupe alkyle en C₁-C₆,
c) phényl-(CH₂)ₒ-, où phényle est non substitué ou substitué une ou deux fois comme décrit aux points 2.1 à 2.12, et o est un entier zéro, 1 ou 2, ou
d) est remplacé par C(O)-O-alkyle en C₁-C₃ et/ou
4.8 deux atomes de carbone dans le cycle forment une partie d'un reste phényle supplémentaire.

3. Composé de formule I selon la revendication 1 ou 2 **caractérisé en ce que**
R¹ représente
1. phényle ou
2. phényle, qui est substitué une fois par un substituant
2.1 alkyle en C₁-C₃, où alkyle est linéaire ou ramifié,
2.2 chlore ou
2.3 -O-méthyle,
R³ représente un atome d'hydrogène ou un groupe méthyle,
R² représente
1. alkyle en C₁-C₄, où alkyle est linéaire ou ramifié et substitué une fois par un substituant phényle, C(O)-OH, halogène, pipéridine ou cyclohexyle, où pipéridine ou cyclohexyle est non substitué ou substitué une ou deux fois par un atome d'halogène ou un groupe C(O)-OH,
2. cyclohexyle,
3. -C (R⁸,R⁷)-C (O)-X, où R⁸ représente un atome d'hydrogène, R⁷ représente un groupe méthyle et X représente un groupe -OH ou -OR⁶, où R⁶ représente un groupe benzyle ou alkyle en C₁-C₃, ou
4. R² et R³ conjointement avec l'atome d'azote auquel ils sont liés forment un reste morpholine, pipéridine, pipérazine ou acide tétrahydro-isoquinoléine-3-carboxylique et où les atomes de carbone dans le cycle sont non substitués ou une ou deux fois substitués par un substituant
4.1 phényle substitué par du chlore ou fluor,
4.2 méthyle,
4.3 =O,
4.4 -C(O)-OH ou
4.5 -C(O)-méthyle,
où dans lequel l'atome d'hydrogène dans le groupe - NH- éventuellement présent dans le cycle est non substitué ou substitué par
a) méthyle,
b) -C(O)-méthyle,
c phényle substitué par du chlore ou
d) est remplacé par C(O)-O-éthyle.

4. Procédé pour la préparation du composé de formule I selon une ou plusieurs des revendications 1 à 3, **caractérisé en ce qu'**on fait réagir
a) une amine de formule III 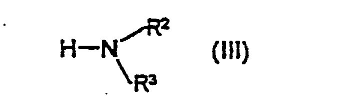 où R² et R³ sont définis comme à la formule I, sur un dérivé d'acide sulfonique de formule IV 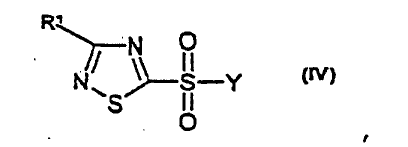 où R¹ est défini comme à la formule I et Y représente un atome d'halogène, un groupe imidazolyle ou -OR¹⁰, où R¹⁰ représente un atome d'hydrogène, un groupe alkyle en C₁-C₆, phényle ou benzyle,
en présence d'une base ou éventuellement d'un agent déshydratant, pour obtenir un composé de formule I, ou on transforme
b) un 5-mercapto-thiadiazole de formule V, 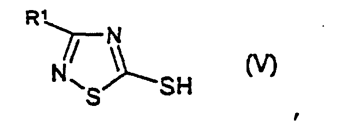 où R¹ possède la signification donnée ci-dessus,
en présence de l'hypochlorure de sodium ou du chlore et d'une amine de formule III, en une sulfénamine de formule VI, 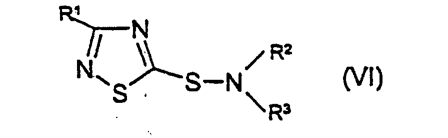 où R¹, R² et R³ possèdent la signification donnée ci-dessus, et on convertit le composé de formule VI en présence d'un agent oxydant tel que des peracides, en particulier l'acide peracétique, l'acide m-chloroperbenzoïque ou le peroxyde d'hydrogène ou le permanganate de potassium, en le composé de formule I, ou on sépare
c) un composé de formule I préparé selon le procédé a) ou b), qui se présente, en raison de sa structure chimique, sous forme énantiomère, par salification par des acides ou bases énantiomères. purs, par chromatographie sur phases stationnaires chirales ou par dérivatisation à l'aide de composés énantiomères chiraux purs, tels que des acides aminés, par séparation des diastéréoisomères ainsi obtenus et par séparation des groupes chiraux auxiliaires, en les formes énantiomères pures, ou
d) le composé de formule I préparé selon le procédé a) ou b) est soit isolé sous forme libre, soit, dans le cas de présence de groupes acides ou basiques, transformé en sels physiologiquement tolérés.

5. Médicaments, **caractérisés par** une teneur efficace en au moins un composé de formule I selon une ou plusieurs des revendications 1 à 3 conjointement avec un véhicule, une matière d'addition et/ou d'autres substances actives et adjuvants tolérés sur le plan pharmaceutique.

6. Utilisation d'au moins un composé de formule I 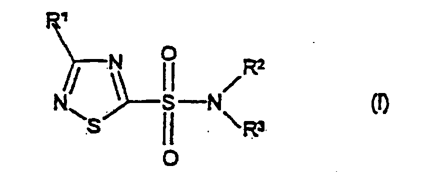 et/ou une forme stéréoisomère du composé de formule I et/ou un sel physiologiquement toléré du composé de formule I, où
R¹ représente
1. phényle,
2. phényle, qui est substitué une à trois fois par des substituants
2.1. alkyle en C₁-C₆, où alkyle est linéaire, cyclique ou ramifié,
2.2. -OH,
2.3. -O-C(O)-alkyle en C₁-C₆,
2.4. -O-alkyle en C₁-C₆,
2.5. méthylènedioxo,
2.6. halogène,
2.7. -CF₃,
2.8. -CN,
2.9. -NO₂,
2.10. -C(O)-OH,
2.11. -C(O)-O-alkyle en C₁-C₆,
2.12. R³(R⁴)N-, où R³ et R⁴ sont identiques ou différents et représentent un atome d'hydrogène ou un groupe alkyle en C₁-C₆, ou
3. un hétéroaromatique pris dans les groupes suivants 3.1 à 3.15, qui est non substitué ou substitué comme décrit au points 2.1 à 2.12
3.1. pyrrole,
3.2. pyrazole,
3.3. imidazole,
3.4. triazole,
3.5. thiophène,
3.6. thiazole,
3.7. oxazole,
3.8. isoxazole,
3.9. pyridine,
3.10. pyrimidine,
3.11. indole,
3.12. benzothiophène,
3.13. benzimidazole,
3.14. benzoxazole ou
3.15. benzothiazole,
R³ représente
1. un atome d'hydrogène ou
2. un groupe alkyle en C₁-C₆,
R² représente
1. un groupe alkyle en C₁-C₇, où alkyle est ramifié ou non ramifié et non substitué ou substitué une ou deux fois comme décrit ci-dessus aux points 2.1 à 2.12 ou par un cycle hétéroalkyle ou par un groupe cycloalkyle en C₃-C₇, où le cycle hétéroalkyle ou le groupe cycloalkyle en C₃-C₇ est non substitué ou substitué une ou deux fois comme décrit ci-dessus aux points 2.1 à 2.12,
2. alcényle en C₂-C₁₀, où alcényle est linéaire ou ramifié,
3. cycloalkyle en C₃-C₇, où cycloalkyle est non substitué ou substitué une ou deux fois comme décrit ci-dessus aux points 2.1 à 2.12,
4. phényle, où phényle est non substitué ou substitué une ou deux fois comme décrit ci-dessus aux points 2.1 à 2.12,
5. (CH₂)ₘ-phényle, où phényle est non substitué ou substitué une ou deux fois comme décrit ci-dessus aux points 2.1 à 2.12, m est un entier 1, 2, 3, 4, 5 ou 6, ou un atome d'hydrogène du reste -(CH₂)ₘ- remplacé par -OH,
6. -(CH₂)ₙ-hétéroaryle, où hétéroaryle est défini comme aux points 3.1 à 3.15 et est non substitué ou substitué comme décrit ci-dessus aux points 2.1 à 2.12 et n est un entier zéro, 1, 2, 3, 4, 5 ou 6,
7. -(alkyle en C₂-C₆) -O-R⁶, où R⁶ représente un atome d'hydrogène, un groupe benzyle, allyle ou alkyle en C₁-C₆ et où le reste alkyle est linéaire ou ramifié,
8 . - (alkyl en C₂-C₆)-C(O)-OR⁶, où R⁶ est défini comme ci-dessus,
9. -C(R⁸,R⁷)-C(O)-X, où R⁸ possède la même signification que R³ ci-dessus, R⁷ représente le reste d'un acide □-aminé de formule II 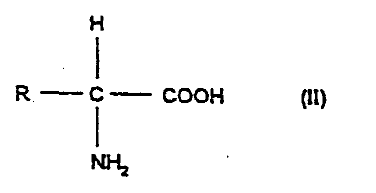 où R dérive d'un acide aminé naturel ou synthétique, ou R⁸ et R⁷, conjointement avec l'atome de carbone auquel ils sont liés, forment un cycle de 4 à 7 chaînons, et X représente un groupe -OH, NHR⁶ ou -OR⁶, où R⁶ est défini comme ci-dessus, ou
10.R² et R³ conjointement avec l'atome d'azote auquel ils sont liés forment un cycle de 4 à 7 chaînons, où éventuellement un des atomes de carbone est remplacé par -O-, -S- ou -NH-, et où un, deux ou trois des atomes de carbone dans le cycle sont non substitués ou substitués une ou deux fois, par des substituants
10.1 alkyle en C₁-C₂,
10.2 phényle,
10.3 -OH,
10.4 =O,
10.5 -C(O)-O-alkyle en C₁-C₆,
10.6 -C(O)-phényle, où phényle est non substitué ou substitué comme décrit ci-dessus aux points 2.1 à 2.12,
10.7 -C(O)-alkyle en C₁-C₆ ou
10.8 -C- (O)-OR³, où R³ représente un atome d'hydrogène ou un groupe alkyle en C₁-C₆, et/ou
10.9 deux atomes de carbone dans le cycle forment une partie d'un reste phényle qui est non substitué ou substitué comme décrit ci-dessus aux points 2.1 à 2.12, ou
où l'atome d'hydrogène dans le groupe -NH-éventuellement présent dans le cycle est non substitué ou substitué par un substituant
a) alkyle en C₁-C₃,
b) -C(O)-R³, où R³ est défini comme ci-dessus,
c) -(CH₂)ₒ-phényle, où phényle est non substitué ou substitué une ou deux fois comme décrit ci-dessus aux points 2.1 à 2.12, et o est l'entier zéro, 1 ou 2,
d) benzoyle, est non substitué ou substitué comme décrit ci-dessus aux points 2.1 à 2.12, ou
e) est remplacé par -C(O)-O-alkyle en C₁-C₆,
pour la préparation de médicaments pour la prévention et la thérapie de maladies qui sont **caractérisées par** l'IL-1β ou par des maladies, qui sont provoquées par la participation l'IL-1β, en particulier de telles où IL-1β est présent concentrations élevées, en comme la leucémie, le choc septique, l'hépatite, les infections par le VIH ou les maladies musculaires et du squelette, telles que la dégradation musculaire, des maladies dégénératives des articulations, telles que des ostéoarthroses, des spondyloses, l'ostéochondrose suite à un traumatisme d'articulations ou à une immobilisation prolongée des articulations suite aux blessures du ménisque ou de la patelle ou des déchirures de ligaments, ou les maladies du tissu conjonctif, comme des collagénoses, des maladies périodontales, les troubles de cicatrisation et en particulier les maladies chroniques de l'appareil moteur telles que des arthrites aiguës et chroniques inflammatoires, immunologiques ou provoquées par le métabolisme, des arthropathies, la polyarthrite, des myalgies et des troubles du métabolisme osseux.

7. Procédé pour la préparation d'un médicament, **caractérisé en ce qu'**on met en une forme d'administration appropriée au moins un composé de formule I selon une ou plusieurs des revendications 1 à 3 avec un véhicule toléré du point de vue pharmaceutique et physiologique et éventuellement d'autres substances actives, matières d'addition ou adjuvants.
